# EUROPEAN PATENT SPECIFICATION

(11) **EP 3 341 081 B1**
(45) Date of publication and mention of the grant of the patent: **11.11.2020**
(21) Application number: 16757886.3
(22) Date of filing: 26.08.2016
(51) Int. Cl.: A61K 35/745, A61K 31/716, A61P 29/00

(54) **USE OF BIFIDOBACTERIUM LONGUM AND AN EXOPOLYSACCHARIDE PRODUCED THEREBY**
VERWENDUNG VON BIFIDOBAKTERIUM LONGUM SOWIE EINEM HIERVON GEWONNENEN EXOPOLYSACCHARID
UTILISATION DE BIFIDOBACTERIUM LONGUM ET EXOPOLYSACCHARIDE PRODUIT ASSOCIÉ

(30) Priority: 27.08.2015 EP 15182694; 27.08.2015 EP 15182695
(43) Date of publication of application: 04.07.2018
(73) Proprietor: PrecisionBiotics Group Limited, Cork T12 N84F (IE)
(72) Inventor: O'MAHONY, Liam, Cork T12 N84F (IE); PLATTNER, Stephan, Cork T12 N84F (IE); ALTMANN, Friedrich, 1140 Wein Vienna (AT); KOSMA, Paul, 1238 Wien Vienna (AT)
(74) Representative: John A. O'Brien & Associates
(86) International application number: PCT/EP2016/070253
(87) International publication number: WO 2017/032897

(56) References cited:
- WO-A1-2008/135959
- WO-A2-2010/055499
- US-A1- 2009 196 921
- WU M H ET AL: "Exopolysaccharide activities from probiotic bifidobacterium: Immunomodulatory effects (on J774A.1 macrophages) and antimicrobial properties", INTERNATIONAL JOURNAL OF FOOD MICROBIOLOGY, ELSEVIER BV, NL, vol. 144, no. 1, 15 November 2010 (2010-11-15), pages 104-110, XP027481268, ISSN: 0168-1605, DOI: 10.1016/J.IJFOODMICRO.2010.09.003 [retrieved on 2010-11-06]

## Description

### Field of the Invention

The present invention relates to *Bifidobacterium longum* NCIMB 41003 for use in the prophylaxis or treatment of allergic airway inflammatory activity. The invention also relates to an exopolysaccharide, and to its use in treating and preventing inflammatory disorders.

### Background of the Invention

The gastrointestinal tract provides a protective interface between the internal environment and the constant challenge from food-derived antigens and from microorganisms in the external environment (Sanderson et al., 1993). The complex ecosystem of the adult intestinal microflora is estimated to harbour 500 different bacterial species. Some of these species are considered potentially harmful because of toxin production, mucosal invasion, or activation of carcinogens and inflammatory responses (Salminen, 1998). However, bacterial strains with health promoting activities have been identified.

Probiotics are beneficial bacteria that exist in the healthy gut microflora and have been defined as a group of live microbial organisms which beneficially affects a host animal by improving its intestinal microbial balance. They consist of "friendly bacteria" which are cultured in laboratory conditions and are then used to restore the balance of the microflora, which has become unbalanced because of, for example stress, illness, or as a result of the use of antibiotics. Importantly, it has been shown that the ingestion of probiotic bacteria can potentially stabilise the immunologic barrier in the gut mucosa by reducing the generation of local proinflammatory cytokines (Isolauri, 1993; Majamaa, 1997). Alteration of the properties of the indigenous microflora by probiotic therapy was shown to reverse some immunologic disturbances characteristic of Crohn's disease (Malin, 1996), food allergy (Majamaa, 1997), and atopic eczema (Isolauri, 2000).

One of the predominant bacterial genus present in the intestinal microflora is *Bifidobacterium.* In the intestines, *Bifidobacterium* ferments sugars to produce lactic acid. The *Bifidobacterium longum* genome codes for many proteins specialised for the catabolism of oligosaccharides, enabling the bacterium to use so-called "nondigestible" plant polymers or host-derived glycoproteins and glycoconjugates. It is thought that *Bifidobacterium's* ability to compete with other gastrointestinal bacteria and occupy a large percentage in the bacterial flora of the gastrointestinal region might be partly due to the large variety of molecules that it is able to use for energy.

While *B. infantis, B. breve,* and *B. longum* are the numerically dominant bacterial group in the intestines of infants, *Bifidobacteria* are said to be only the 3rd or 4th largest group of bacteria in adults (and only 3-6% of adult fecal flora). The number of these bacteria actually decline in the human body with age. In infants who are breast-fed, *Bifidobacteria* constitute about 90% of their intestinal bacteria; however, this number is lower in formula-fed infants. When breast-fed infants' diets are changed to cows milk and solid food, *Bifidobacteria* are joined by rising numbers of other bacteria found in the human body such as *Bacteroides* and *Streptococci lactobacilli.*

Bifidobacteria have been shown to play a role in the modulation of the immune system. *B. breve* is thought to release metabolites exerting an anti-TNF effect capable of crossing the intestinal barrier. Mucosal inflammation in interlukin-10 (IL-10) deficient mice has been reported to be reduced by feeding the subject animals a preparation of lactic acid bacteria (Madsen, K et al., 1997; O'Mahony et al., 2001; McCarthy et al., 2004). WO 00/41168 discloses a strain of *Bifidobacterium* isolated from resected and washed human gastrointestinal tract which is significantly immunomodulatory following oral consumption in humans.

Scientific research indicates an increasing incidence of illness which may be caused by deficient or compromised microflora (natural microbial resident population of the digestive system) such as gastrointestinal tract (GIT) infections, constipation, irritable bowel syndrome (IBS), inflammatory bowel disease (IBD), Crohn's disease and ulcerative colitis, food allergies, antibiotic-induced diarrhoea, cardiovascular disease and certain cancers such as colorectal cancer. Evidence indicates that following treatment with a single *Bifidobacterium* strain, IBS symptom severity is reduced (Whorwell et al., 2006). Efficacy is associated with modulation of systemic immune responses indicating that the mechanism of action, in part, is immune mediated (O'Mahony et al., 2005). The present invention describes a compound isolated from *Bifidobacterium* that replicates the immunomodulatory activity of *Bifidobacterium in vitro.*

WO2008/135959A discloses an exopolysaccharide isolated from *Bifidobacterium* strain NCIMB 41003.

WO2010/055499A discloses various *Bifidobacterium longum* strains with specific reference to *Bifidobacterium longum* strain NCIMB 41003.

US2009/196921A describes compositions including a probiotic strain.

Wu M H et al: "Exopolysaccharide activities from probiotic bifidobacterium: Immunomodulatory effects (on J774A. 1 macrophages) and antimicrobial properties", International Journal of Food Microbiology, Elsevier BV, NL, vol. 144, no. 1, 15 November 2010 (2010-11-15), pages 104-110, XP027481268, ISSN: 0168-1605, DOI: 10.1016/J.IJFOODMICRO.2010.09.003 discloses that exopolysaccharides isolated from *Bifidobacterium* strains including *Bifidobacterium* strain BCRC 14634 might be useful.

### Statements of Invention

According to the invention there is provided *Bifidobacterium longum* NCIMB 41003 for use in the prophylaxis or treatment of asthma, chronic obstructive pulmonary disease (COPD), eosinophilic COPD, infection-associated inflammation, allergen-induced inflammation, allergic rhinitis or chronic rhinosinusitis.

The invention also provides a polysaccharide comprising the structure 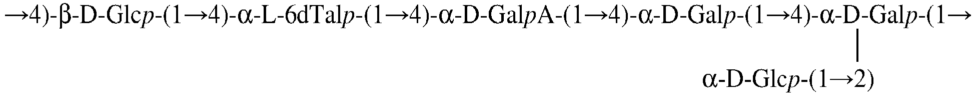 for use in asthma, chronic obstructive pulmonary disease (COPD), eosinophilic COPD, infection-associated inflammation, allergen-induced inflammation, allergic rhinitis or chronic rhinosinusitis.

At least one of the subunits of the polysaccharide may be O-acetylated.

Also described is an exopolysaccharide comprising the structure

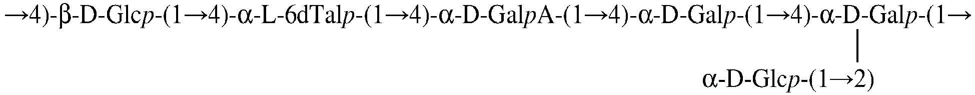

At least one of the subunits of the polysaccharide may be O-acetylated.

The polysaccharide may be obtainable from a *Bifidobacterium* strain.

The polysaccharide may be obtained from a *Bifidobacterium longum* strain.

The polysaccharide may be obtained from *Bifidobacterium longum* NCIMB 41003.

The polysaccharide may comprise a chain of more than two units defined.

The polysaccharide may have a molecular weight of up to 10 MDa.

Also described is an expression delivery system for delivery of an exopolysaccharide as defined to inflamed organs or tissue, the delivery system comprising a strain of *Bifidobacterium longum.* The *Bifidobacterium longum* may be *Bifidobacterium longum* NCIMB 41003.

The polysaccharide may be for use in therapy. The polysaccharide may be for treating or preventing undesirable inflammatory activity. The undesirable inflammatory activity may be gastrointestinal inflammatory activity. The gastrointestinal inflammatory activity may be Crohns disease, ulcerative colitis, irritable bowel syndrome, pouchitis, post infection Crohns disease, ulcerative colitis, irritable bowel syndrome, pouchitis, post infection colitis, *Clostridium difficile* associated diarrhoea, Rotavirus associated diarrhoea or post infective diarrhoea.

The undesirable inflammatory activity may be due to allergic inflammatory activity. The allergic inflammatory activity may be in one or more of multiple body organs, including the gastrointestinal tract, the nose, the eyes, the lungs, the skin, or systemically resulting in multiple tissue responses culminating in anaphylaxis.

The polysaccharide of the invention may be used for treating or preventing undesirable airway inflammatory activity. The airway inflammatory activity may be asthma, chronic obstructive pulmonary disease (COPD), eosinophilic COPD, infection-associated inflammation, allergen-induced inflammation, allergic rhinitis or chronic rhinosinusitis.

Also described is the use of a polysaccharide as defined in the preparation of a medicament for treating or preventing undesirable inflammatory activity. The undesirable inflammatory activity is undesirable gastrointestinal inflammatory activity.The gastrointestinal inflammatory activity is Crohn's disease, ulcerative colitis, irritable bowel syndrome, pouchitis, post infection Crohn's disease, ulcerative colitis, irritable bowel syndrome, pouchitis, post infection colitis, *Clostridium difficile* associated diarrhoea, Rotavirus associated diarrhoea or post infective diarrhoea.

The undesirable inflammatory activity may be rheumatoid arthritis.

Also described is the use of a polysaccharide as defined in the preparation of a medicament for treating or preventing autoimmune disorders.

Also provided is the use of a polysaccharide as defined in the preparation of a medicament for treating or preventing allergic inflammatory activity. The allergic inflammatory activity may be in one or more of multiple body organs, including the gastrointestinal tract, the nose, the eyes, the lungs, the skin, or systemically resulting in multiple tissue responses culminating in anaphylaxis.

The undesirable inflammatory activity may be undesirable airway inflammatory activity.
The airway inflammatory activity is asthma, chronic obstructive pulmonary disease (COPD), eosinophilic COPD, infection-associated inflammation, allergen-induced inflammation, allergic rhinitis or chronic rhinosinusitis.

Also described is a pharmaceutical composition comprising a polysaccharide of the invention and a pharmaceutically acceptable carrier.

Also described is a foodstuff comprising a polysaccharide of the invention. The foodstuff in some cases is one or more selected from the group comprising: yogurts, cereals, beverages.

### Brief Description of the Figures

The invention will be more clearly understood from the following description thereof given by way of example only in which:-
Fig. 1: Size exclusion chromatography of *Bifidobacterium longum* NCIMB 41003 (35624™) EPS (solid line) in comparison with a dextran standard of 1.1 MDa Mw (dotted line);
Fig. 2: PMP-labelled monosaccharides were separated on a reversed-phase column with UV detection revealing glucose, galactose, a small amount of galacturonic acid and additional compounds unidentified using this method;
Fig. 3: Analysis of the monosaccharide composition of *Bifidobacterium longum* NCIMB 41003 (35624™) EPS by HPLC of anthranilic acid-labeled sugars using the phosphate buffer system. The upper panel shows the standard run, the panel below the EPS sample. The identity of the GalA-Gal peak was inferred from experiments with a volatile buffer system (see Fig. 4). Amino sugars, neutral hexoses, hexuronic acids and deoxy-hexoses are labelled in different colours in the standard run. In the lower panel, peaks identified only by subsequent experiments are labelled with smaller font;
Fig. 4: Analysis of the monosaccharide composition of *Bifidobacterium longum* NCIMB 41003 (35624™) EPS by HPLC of anthranilic acid-labeled sugars using a volatile buffer system that allowed MS analysis of collected peaks. Off-line MS analysis identified one unknown peak as aldobiuronic acid and the second one as deoxy-hexose;
Fig. 5: Linkage analysis by GC-MS of partially permethylated alditol acetates. A: Result after pre-hydrolysis with 50 mM TFA for 4h at 80°C. B: Result after prehydrolysis with 250 mM TFA. Note the occurrence of an additional peak representing 2-substituted galactose. Galacturonic acid is not visible with this method. The insert in A shows the electron impact fragment spectrum of the peak at 18.8 min;

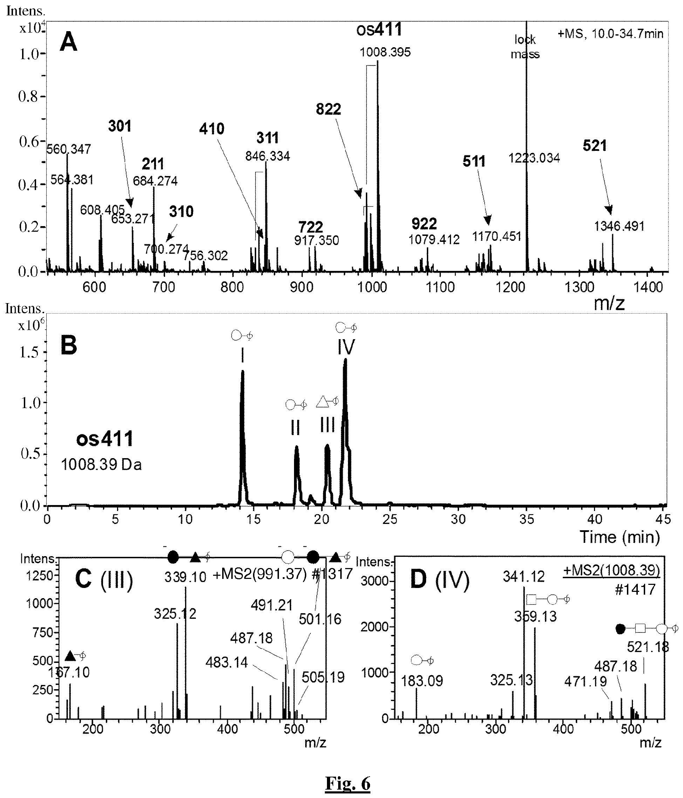

Fig. 6: Reduced, underivatized glycans obtained by mild acid hydrolysis of *Bifidobacterium longum* NCIMB 41003 (35624™) EPS. A. Wide range sum spectrum of the PGC-LC-ESI-MS analysis of partially hydrolysed EPS. The composition is indicated by figures giving the number of hexoses, hexuronic acids and deoxyhexoses. Many glycans were observed as H+ only and H+/NH4+ ions. B. Selected ion chromatogram for the composition 4-1-1 (H+/NH4+ ion). The reducing sugar as revealed by MS/MS is shown on top of each peak. C. Parts of the MS/MS spectra of isomers III and IV identifying their reducing end. Isomers I and II terminate in hexose just like IV. Due rearrangements conclusions other than about the reducing end sugar could not be made;
Fig. 7: Reduced and deutero-permethylated os211 was analyzed by MALDI-TOF MS and the tetrasaccharide ion at 927.2 Da was subjected to laser-induced dissocation. The sequence of the y2 ion was clear from ESI-MS/MS of the unmethylated glycan, which revealed hexose as the reducing sugar. The sequence of the b2 ion was inferred from GC-MS analysis of partially methylated alditol acetates, which showed 4-linked deoxyhexose (data not shown) as well as from the MS/MS pattern of os311a and os411a;
Fig. 8: Reduced os311 fragments were separated by PGC-LC, collected deutero-permethylated and then analyzed by MALDI-TOF MS of mass 1140.3. The y2 fragment in os311a implies the position of the branching in this fragment. The inserts depict the inferred fragment structure;
Fig. 9: Reduced os411 fragments were separated by PGC-LC, collected deutero-permethylated and then analyzed by MALDI-TOF/TOF MS of mass 1140.3. The y2 fragment in os411a implies the position of the branching in this fragment. The inserts depict the inferred fragment structure;
Fig. 10: Monosaccharides were analysed by GC-MS after derivatization with L-cysteine methyl ester and trimethylsilylation. The retention time of L-glucose was inferred from literature [Hara S. et al (1987)];
Fig. 11 : 600 MHz ¹H NMR spectrum of the acid-treated exopolysaccharide (D₂O, 338 K) from *B. infantis* bif624. Part of the high-field region is displayed in the insert;
Fig. 12 : Expansion plot of the 150 MHz ¹³C NMR spectrum of the acid-treated *Bifidobacterium longum* NCIMB 41003 (35624™) exopolysaccharide;
Fig. 13 : Selected region of the multiplicity-edited, gradient-enhanced ¹H, ¹³C-HSQC NMR spectrum (600 MHz) of the acid-treated *Bifidobacterium longum* NCIMB 41003 (35624™) exopolysaccharide. The letters denote the residues as given in Fig. 18. Resonances from anomeric, substitution positions and resolved signals are annotated;
Fig. 14 : Selected region of the ¹H, ¹³C-HSQC-TOCSY NMR spectrum (600 MHz) of the acid-treated *Bifidobacterium longum* NCIMB 41003 (35624™) exopolysaccharide. Arabic numerals before and after oblique stroke denote carbons and protons, respectively;
Fig. 15 : 600 MHz ¹H NMR spectrum of the tetrasaccharide alditol fragment os211;
Fig. 16 : Selected region of the ROESY-spectrum of the acid-treated exopolysaccharide from *Bifidobacterium longum* NCIMB 41003 (35624™) . Inter-residue cross-peaks are denoted in bold. The letters denote the residues as given in Fig. 18 and arabic numerals refer to the respective protons;
Fig. 17 : Synthesis of 6-deoxy-L-talose;
Fig. 18: Structure of the *Bifidobacterium longum* NCIMB 41003 (35624™) exopolysaccharide. Scheme **A** depicts the chemical structure of the repeating unit; letters A to F correspond to the subunits described in Table 1. Scheme **B** shows the same structure from a different angle. Scheme C represents the EPS structure in conventional IUPAC code;
Fig. 19: MDDC secretion of proinflammatory cytokines in response to *Bifidobacterium longum* NCIMB 41003 (35624™) exopolysaccharide;
Fig. 20: MDDC secretion of the regulatory cytokine IL-10 is TLR-2 dependent;
Fig. 21: MDDC secretion of cytokines in response to *Bifidobacterium longum* NCIMB 41003 (35624™) and the isogenic exopolysaccharide-deficient mutant sEPSneg;
Fig. 22: PBMC secretion of cytokines in response to *Bifidobacterium longum* NCIMB 41003 (35624™), the isogenic exopolysaccharide-deficient mutant sEPSneg and the exopolysaccharide-complemented sEPScomp strain;
Fig. 23: PBMC secretion of cytokines in response to the isogenic exopolysaccharide-deficient mutant sEPSneg, with or without the addition of isolated exopolysaccharide;
Fig. 24: Murine weight loss and colitis disease severity scores in response to *Bifidobacterium longum* NCIMB 41003 (35624™), the isogenic exopolysaccharide-deficient mutant sEPSneg and the exopolysaccharide-complemented sEPScomp strain;
Fig. 25: Colon:body weight ratio and photographs of colons from SCID mice administered *Bifidobacterium longum* NCIMB 41003 (35624™), the isogenic exopolysaccharide-deficient mutant sEPSneg and the exopolysaccharide-complemented sEPScomp strain;
Fig. 26: Intracellular cytokine staining of lymphocytes from mesenteric lymph nodes of SCID mice administered *Bifidobacterium longum* NCIMB 41003 (35624™), the isogenic exopolysaccharide-deficient mutant sEPSneg and the exopolysaccharide-complemented sEPScomp strain;
Fig. 27: *Bifidobacterium longum* NCIMB 41003 (35624™) cells significantly reduce the percentage of eosinophils within the lung of OVA allergic mice; and
Fig. 28: *Bifidobacterium longum* NCIMB 41003 (35624™) Exopolysaccharide (EPS) significantly reduces the percentage of eosinophils within the lung of OVA allergic mice;
Fig. 29: *Bifidobacterium longum* NCIMB 41003 (35624™) Exopolysaccharide (EPS) significantly reduces CCL11 gene expression, with a trend for reduced CCR3 gene expression, within the lung of OVA allergic mice;
Fig. 30: *Bifidobacterium longum* NCIMB 41003 (35624™) Exopolysaccharide (EPS) significantly reduces Ym-1 gene expression within the lung of OVA allergic mice;
Fig. 31: *Bifidobacterium longum* NCIMB 41003 (35624™) Exopolysaccharide (EPS) significantly increases lung Foxp3 gene expression;
Fig. 32: *Bifidobacterium longum* NCIMB 41003 (35624™) Exopolysaccharide (EPS) significantly increases the percentage of lung lymphocytes expressing Foxp3;
Fig. 33: Blockade of TLR-2 reduces the ability of *Bifidobacterium longum* NCIMB 41003 (35624™) Exopolysaccharide (EPS) to suppress eosinophil recruitment into the lung; and
Fig. 34: The loss of IL-10 in IL-10-deficient mice reduces the ability of *Bifidobacterium longum* NCIMB 41003 (35624™) Exopolysaccharide (EPS) to suppress eosinophil recruitment into the lung.

### Detailed description

Various preferred features and embodiments of the present invention will now be described by way of non-limiting example.

The practice of the present invention will employ, unless otherwise indicated, conventional techniques of chemistry, microbiology and immunology, which are within the capabilities of a person of ordinary skill in the art. Such techniques are explained in the literature.

We have identified an exopolysaccharide with immuomodulatory properties from *Bifidobacterium longum* NCIMB 41003 (35624™).

### Exopolysaccharide

The present invention relates to an exopolysaccharide biosynthesised by *Bifidobacterium longum* NCIMB 41003 (35624™). Polysaccharides are synthesised by a wide variety of microorganisms and are usually repeating sugar units which remain associated with the cell surface or are secreted or both. They play a role in both cellular stress responses or can contribute to the virulence of a pathogen. Recently, an immunomodulatory role for *Bacteroides fragilis* exopolysaccharide has been demonstrated (Mazmanian et al., 2005).

### Inflammation

Inflammation is a local response to cellular injury that is marked by capillary dilatation, leukocytic infiltration, redness, heat, pain, swelling, and often loss of function. Control of the inflammatory response is exerted on a number of levels (for review see Henderson B., and Wilson M. 1998. The controlling factors include cytokines, hormones (e. g. hydrocortisone), prostaglandins, reactive intermediates and leukotrienes.

Cytokines are low molecular weight biologically active proteins that are involved in the generation and control of immunological and inflammatory responses, while also regulating development, tissue repair and haematopoiesis. They provide a means of communication between leukocytes themselves and also with other cell types. Most cytokines are pleiotrophic and express multiple biologically overlapping activities. Cytokine cascades and networks control the inflammatory response rather than the action of a particular cytokine on a particular cell type (Arai KI, et al., 1990). Waning of the inflammatory response results in lower concentrations of the appropriate activating signals and other inflammatory mediators leading to the cessation of the inflammatory response. Tumor necrosis factor alpha (TNFα) is a pivotal proinflammatory cytokine as it initiates a cascade of cytokines and biological effects resulting in the inflammatory state. Therefore, agents which inhibit TNFα are currently being used for the treatment of inflammatory diseases, e. g. infliximab.

Pro-inflammatory cytokines are thought to play a major role in the pathogenesis of many inflammatory diseases, including inflammatory bowel disease (IBD). Current therapies for treating IBD are aimed at reducing the levels of these proinflammatory cytokines. The exopolysaccharide of the present invention may have potential application in the treatment of inflammatory disorders. This may be achieved, for example, by increasing the concentration of non-inflammatory cytokines such as, but not limited to IL-10, and/or decreasing the concentration of inflammatory cytokines.

### Inflammatory Bowel Disease

Inflammatory bowel disease (IBD) is characterised by a chronic relapsing intestinal inflammation. IBD is subdivided into Crohn's disease and ulcerative colitis phenotypes. Crohn's disease may involve any part of the gastrointestinal tract, but most frequently the terminal ileum and colon. In approximately 10% of cases confined to the rectum and colon, definitive classification of Crohn's disease or ulcerative colitis cannot be made and are designated 'indeterminate colitis.' Both diseases include extra-intestinal inflammation of the skin, eyes, or joints.

Crohn's disease and ulcerative colitis are commonly classified as autoimmune diseases as both illnesses are marked by an abnormal response by the body's immune system resulting in chronic inflammation in the lining of the intestines. The prevalence of inflammatory bowel disease is increased in individuals with other autoimmune diseases, particularly ankylosing spondylitis, psoriasis, sclerosing cholangitis, and multiple sclerosis.

### Crohn's disease

Crohn's disease is a chronic disorder that causes inflammation of the digestive or gastrointestinal wherein the immune system attacks the intestine.

Although Crohn's disease most commonly affects the end of the ileum and the beginning of the colon, it may involve any part of the gastrointestinal tract. Bowel inflammation is transmural and discontinuous; it may contain granulomas or be associated with intestinal or perianal fistulas. The CARD15 gene and an allele of the ABCB1 gene are thought to be associated with susceptibility to Crohn's disease.

### Ulcerative Colitis

Ulcerative colitis is a disease that causes inflammation and sores in the lining of the large intestine. It is a nonspecific chronic inflammatory disease affecting the bowel. Ulcers form and bleed in places where the inflammation has killed the cell lining. In contrast to Crohn's disease, the inflammation is continuous and limited to rectal and colonic mucosal layers; fistulas and granulomas are not observed.

Both genetic and environmental factors seem to be important in its etiology. Fuss et al. examined lamina propria T cells from patients with ulcerative colitis and found that they produced significantly greater amounts of IL13 and IL5 than control or Crohn's disease cells and little IFN-gamma. They concluded that ulcerative colitis is associated with an atypical Th2 response mediated by nonclassic NKT cells that produce IL13 and have cytotoxic potential for epithelial cells.

### Pouchitis

Chronic and/or acute inflammation of the ileal reservoir, so-called "pouchitis", is a frequently observed long-term complication of the ileo-anal pouch anastomosis. In ulcerative colitis patients, the prevalence of pouchitis varies from less than 10% to higher than 40%. The definition of "pouchitis" includes clinical symptoms, macroscopic inflammatory lesions at endoscopy and histological evidence of intense acute inflammation of the reservoir mucosa.

### Clostridium difficile associated diarrhoea

*Clostridium difficile* is an anaerobic, gram-positive spore forming bacillus first isolated in 1935 from faecal flora of healthy neonates. It was not until 1978 that its association with antibiotic induced pseudomembranous colitis (PMC) was established. Almost all antibiotics have been linked with *C*. *difficile* diarrhoea and colitis, including vancomycin and metronidazole (which are used for its treatment) and cancer chemotherapy. The frequency of association is related to frequency of use, the route of administration and the impact of that antibiotic on the colonic microflora.

### Irritable bowel syndrome

Irritable bowel syndrome (IBS) is a chronic disorder that interferes with the normal functions of the large intestine (colon). It is characterised by a group of symptoms - crampy abdominal pain, bloating, constipation, and diarrhoea.

IBS causes a great deal of discomfort and distress, but it does not permanently harm the intestines and does not lead to intestinal bleeding or to any serious disease such as cancer. Signs and symptoms of IBS vary widely from one person to another and often occur with many other diseases.

### COPD

Chronic obstructive pulmonary disease (COPD) is a common lung disease. There are two main forms of COPD, Chronic bronchitis, which involves a long-term cough with mucus and Emphysema, which involves damage to the lungs over time. Most people with COPD have a combination of both conditions.COPD is the third leading cause of death in the USA. In fact COPD is the only major cause of death whose incidence is on the increase and is expected to be the third leading cause of death in the developed world by 2030 (exceeded only by heart disease and stroke). It results from inflammation induced damage (including Th17-associated inflammatory responses) of the airways causing chronic bronchitis and/or emphysema. The condition leads to progressive irreversible airflow obstruction with shortness of breath, coughing, and excessive sputum (mucus) production. A survey of 125 European pulmonologists shows a low diagnosis rate and large untreated population, particularly among mild patients (Datamonitor, 2011). This prevalence, together with the limited efficacy of current COPD drugs, represents a major medical need. The suppression of TNF alpha and IL-17, coupled with the induction of IL-10, as described below for the EPS-secreting strain and the isolated EPS, would be expected to dampen proinflammatory damaging activities within the lungs of COPD patients.

### Inflammatory Skin Disease

Inflammatory skin diseases include but are not limited to Psoriasis, Eczema (also known as Atopic Dermatitis), Rosacea, and Acne. One common feature of these diseases and the underlying inflammation is the dysregulation of the cytokines IL-10 and TNF-alpha.

### Atopic dermatitis

(AD; a type of eczema) is an inflammatory, chronically relapsing, non-contagious and pruritic skin disorder. It has been given names like "prurigo Besnier," "neurodermitis," "endogenous eczema," "flexural eczema," "infantile eczema," and "prurigo diathsique".

Atopic dermatitis often occurs together with other atopic diseases like hay fever, asthma and conjunctivitis. It is a familial and chronic disease and its symptoms can increase or disappear over time. Atopic dermatitis in older children and adults is often confused with psoriasis. Atopic dermatitis afflicts humans, particularly young children; it is also a well-characterized disease in domestic dogs.

The invention relates to the prophylaxis and/or treatment of such inflammatory skin diseases. Inflammatory skin diseases include but are not limited to Psoriasis, Eczema (also known as Atopic Dermatitis), Rosacea, and Acne. One common feature of these diseases and the underlying inflammation is the dysregulation of the cytokines IL-10 and TNF-alpha, which are both influenced by the EPS-secreting bacterium and the isolated EPS.

### Psoriasis

Psoriasis is a chronic, non-infectious inflammatory disease that affects mainly the skin. It is currently suspected to be autoimmune in origin. It commonly causes red, scaly patches to appear on the skin, although some patients have no dermatological symptoms. The scaly patches caused by psoriasis, called psoriatic plaques, are areas of inflammation and excessive skin production. Psoriasis can also cause inflammation of the joints, which is known as ***psoriatic arthritis.*** Ten to fifteen percent of people with psoriasis have psoriatic arthritis.

Various environmental factors have been suggested as aggravating to psoriasis including stress, withdrawal of systemic corticosteroid, excessive alcohol consumption, and smoking but few have shown statistical significance. There are many treatments available, but because of its chronic recurrent nature psoriasis is a challenge to treat.

T cells (which normally help protect the body against infection) become active, migrate to the dermis and trigger the release of cytokines (IL-17 and tumor necrosis factor-alpha TNFα, in particular) which cause inflammation and the rapid production of skin cells. It is not known what initiates the activation of the T cells. The immune-mediated model of psoriasis is supported by the observation that immunosuppressant medications can clear psoriasis plaques. Early results of treatment of psoriatic arthritis and psoriasis with TNF neutralising agents are encouraging (P J Mease Ann Rheum Dis 2002;61:298-304 doi:10.1136/ard.61.4.298). The EPS-expressing bacterial strain reduces IL-17-secreting lymphocytes in the murine model described below, while the EPS-deficient bacterial strain loses this effect, suggesting that the EPS suppresses abberent TH17 responses. In addition, the isolated EPS suppresses TNF-alpha secretion in vitro.

Interleukin-10 is dysregulated in and considered to be a key cytokine in psoriasis, prompting efforts toward IL-10 therapy (J. Clin. Invest. 1998. 101:783-794.). IL-10 promoter polymorphisms influence disease severity and course in psoriasis (Kingo et al. Genes Immun. 2003 Sep; 4(6):455-7). The isolated EPS induces IL-10 in vitro, which would be expected to dampen skin inflammatory responses such as those observed in psoriasis patients.

### Joints

The EPS described herein may also be used in treating joints in mammals such as humans. The polysaccharide in some cases may be formulated in a composition for topical application such as in the form of an ointment, lotion, cream, gel powder or spray. Alternatively it can be formulated in a form suitable for injection directly into a joint and in particular into synovial fluid surrounding a joint. In this way the polysaccharide may be used for prophylaxis or treatment of joint conditions such as osteoarthritis or the pain associated therewith. Such compositions and methods may be used in association with other active ingredients such as hyaluronic acid and/or chondroitin sulfate and/or glucosamine, for example as described in US2016/0206649A, the entire contents of which are herein incorporated by reference. The *Bifidobacterium longum* which produces the EPS may also be used to achieve similar effects. The *Bifidobacterium longum* for this use may be in a form for oral consumption such as a food or pharmaceutical which may, for example, be in the form of a tablet, powder or capsule. The *Bifidobacterium longum* may be in the form of viable or non-viable cells. The suppression of TNF alpha and IL-17, coupled with the induction of IL-10, as described below for the EPS-secreting strain and the isolated EPS, would be expected to dampen proinflammatory damaging activities within the joints.

### Wounds

The EPS described herein may also be used for treating wounds in mammals such as humans to promote wound healing. The polysaccharide in such cases may be formulated in a composition for application to a wound such as in the form of an ointment, powder, paste, gel or spray or as part of or incorporated into a matrix or a dressing. Such compositions and methods may be used in association with other therapeutic agents including a chitosan and/or an antimicrobial agent, for example as described in US2015/0119358A, the entire contents of which are incorporated herein by reference. The *Bifidobacterium longum* which produces the EPS may also be used to achieve similar effects. The *Bifidobacterium longum* for this use may be in a form for oral consumption such as a food or pharmaceutical which may, for example, be in the form of a tablet, powder or capsule. The *Bifidobacterium longum* may be in the form of viable or non-viable cells. The suppression of TNF alpha and IL-17, coupled with the induction of IL-10, as described below for the EPS-secreting strain and the isolated EPS, would be expected to dampen proinflammatory damaging activities within the skin and promote optimal wound healing.

### Exopolysaccharide Delivery System

The invention also provides a delivery system for delivery of an exopolysaccharide as defined to inflamed organs or tissue, the delivery system comprising a strain of *Bifidobacterium longum.* The *Bifidobacterium longum* may be *Bifidobacterium longum* NCIMB 41003. The delivery system may be used to deliver the polysaccharide for prophylaxis or treatment of any of the diseases and conditions as described herein.

### Other active ingredients

It will be appreciated that the exopolysaccharide of the present invention may be administered prophylactically or as a method of treatment either on its own or with other probiotic and/or prebiotic materials. In addition, the exopolysaccharide may be used as part of a prophylactic or treatment regime using other active materials such as those used for treating inflammation or other disorders, especially those of the gastrointestinal tract. Such combinations may be administered in a single formulation or as separate formulations administered at the same or different times and using the same or different routes of administration.

### Pharmaceutical compositions

A pharmaceutical composition is a composition that comprises or consists of a therapeutically effective amount of a pharmaceutically active agent. It preferably includes a pharmaceutically acceptable carrier, diluent or excipients (including combinations thereof). Acceptable carriers or diluents for therapeutic use are well known in the pharmaceutical art, and are described, for example, in Remington's Pharmaceutical Sciences. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), propellants(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in a mixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner. Intranasal administration can be accomplished using a nasal spray, nasal wash solution or direct application within the nose. Administration to the lung could be in the form of a dry powder, inhaled using an inhaler device. In some cases the formulation is in the form of an aerosol. The aerosol may be a solution, suspension, spray, mist, vapour, droplets, particles, or a dry powder, for example, using a method dose inhaler including HFA propellant, a metered dose inhaler with non-HFA propellant, a nebulizer, a pressurized can, of a continuous sprayer.

The sEPS produced by 35624 may comprise a repeating subunit that consists of six monosaccharides, of which one is an epimer of glucuronic acid, one or two others are either D-fucose or 6-deoxy-talose, and some of which may be O-acetylated.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by both routes.

### Purification of exopolisaccharide

*Bifidobacterium longum* NCIMB 41003 (35624™) is described in WO 00/42168, the entire contents of which are incorporated herein by reference. The strain was deposited at the NCIMB on January 13, 1999.

An Eps has been isolated previously from *Bifidobacterium* 35264 (NCIMB 41003) and is described in WO 2008/135959 A1. We have modified the extraction procedure and we isolated an EPS with a different structure. The new method reduces the centrifugation speed from 40000g to 20000g. By using a lower centrifugation speed the large EPS molecules stay in solution and are not precipitating during the centrifugation. We have also omitted the TCA precipitation as the low pH could hydrolyse the EPS. Additionally we have complemented the MRS media with a higher COH concentration (>2%) in order to stimulate a higher EPS production. These changes led to the isolation of a novel exoplysacharide from *Bifidobacterium longum* 35624 which has not been described previously.

In the following we describe a process for providing an exopolysaccharide (EPS) from *Bifidobacterium longum* NCIMB 41003 (35624™) by using two intrinsic physio-chemical properties of this specific exopolisaccharide (A,B).
A) We have identified a novel process to purify and concentrate the described polysaccharide. It was found that the exopolysaccharide, if present at sufficient concentration, aggregates on the surface of ethanol or similar alcohol solutions. The EPS aggregation at the surface can be collected and separated from the rest without centrifugation. The aggregated compound can be solubilized by transferring it into PBS or water. This procedure applied a single time or several times in a row, allows a simple separation of the polysaccharide from contaminating substances without the need of centrifugation
B) Additionally, it was found that the described exopolysaccharide is not binding to typical reverse phase columns when present in the water phase. This finding allows a further purification of the EPS solved in water by applying the EPS solution on typical reverse phase columns and by collecting the flow-through fraction. Contaminating compounds are bound to the column.

By using these intrinsic properties the following procedures can be used to purify the described EPS from 35624 cultures of different sources. The here described EPS can be obtained from the growth media as secreted EPS or can be obtained from the bacterial cell wall as capsular EPS. Both methods were used to generate the exopolysaccharide described in this application.

### The secreted EPS

Secreted EPS are high molecular weight sugar compounds which are released by the bacterial cells into the growth medium during the fermentation. The growth media can be separated from bacterial cells by standard centrifugation or filtration. The described EPS can be purified from the growth medium by the addition of ethanol or similar alcohols to a final concentration of 20-95%. The here described EPS in the growth media will precipitate, aggregate and eventually appear at the surface of the solution. Circular movements of the solution can be applied to center the aggregations at the surface where it can be collected and separated from the solution without centrifugation.

### Capsular EPS

Capsular EPS is considered as high molecular weight sugar compounds which are covalently or non-covalently bound to the outer cell membrane. The bound EPS can be separated by NaOH treatment of the cells using known methods. It was found that the capsular EPS from *Bifidobacterium longum* NCIMB 41003 (35624™) can be further processed in the same way as the secreted EPS described under 1).

The NaOH treated cells are removed by centrifugation and filtration and the solution is neutralized with a monovalent acid like HCl. It was found that by addition of ethanol or a similar alcohol to a final concentration of 20-95% the here described EPS in the growth media will precipitate, aggregate and eventually appear at the surface of the solution. Circular movements of the solution can be applied to center the aggregations at the surface where it can be collected and separated from the solution without centrifugation.

### Culture preparations of Bifidobacterium longum NCIMB 41003 (35624™)

The bacterium can be grown under different conditions.

A common way to grow Bifidobacteria is the use of a pH controlled fermentation media suitable for the strain. A constant CHO concentration from 2-15 % or higher or a constant feeding of CHO solutions can be used to supply an adequate amount of Carbon needed for EPS synthesis. The bacterium can also be grown as a bacterial lawn or in single colonies on standard agar plates using standard or modified Bifidobacterium agar medium and cells can be harvested by scrapping or washing the cells from the media. The growth of the cells on plates provides a cell suspension with a lower amount of contaminants compared to liquid fermentations where a higher carryover of media components can be expected.

The starting material may be a freeze-dried powder of *Bifidobacterium longum* NCIMB 41003 (35624™). The powder can be re-suspended in aqueous solutions and capsular or secreted EPS can be extracted as described above.

### EXAMPLES

### Example 1

### Bifidobacterium longum NCIMB 41003 (35624™) was grown as single colonies on plates. Secreted EPS was isolated by ethanol precipitation and applied twice to a reverse phase column

*Bifidobacterium longum* NCIMB 41003 (35624™) was grown on Modified Rogosa Agar medium prepared from the following components per litre: peptone from casein 10 g (Merck), yeast extract 2.5 g (Merck), Tryptose 3 g (Oxoid), K₂HPO₄ 3 g (Sigma), KH₂PO₄ 3 g (Sigma), ammonium citrate tribasic 3 g (Sigma), sodium pyruvate 0.2 g (Sigma), Tween 80 1 ml, MgSO4^{∗}7H2O 0.575 g, MnSO4^{∗}4H2O 0.12 g, FeSO4^{∗}7H2O 0.034 g, technical agar 15 g (pH 6.8 adjusted with NaOH before autoclaving). A 30% glucose solution was autoclaved separately and added together with sterile filtrated L-cysteine hydrochloride solution to the media to a final concentration of 3% and 0.05 %, respectively. The media was poured into petri dishes and dried under laminar flow for 30 min.

*Bifidobacterium longum* NCIMB 41003 (35624™) in form of a freeze-dried powder with a bacterial count of 1.0E11 CFU/g was used as inoculum. The powder was serially diluted in PBS to 1E-8 and 300 µl of the solution with approximately 300 CFU were spread plated on modified Rogosa Agar plates.

The plates were incubated at 37°C under anaerobic conditions for 60 h. Cells were harvested using cell scrapers and resuspended for 2 h under constant steering in PBS containing Ribonuclease A (1 µg/ml) and Deoxyribonuclease I (5 µg/ml Sigma).

The cells were centrifuged for 30 min at 4°C and 20000 g (Sorval RC6 plus) and the supernatant was filtered through 0.45 µm syringe filters. The EPS containing solution was poured into 3 volumes of chilled ethanol, gently stirred for 30 seconds and incubated at 4°C for 2 h. The precipitating EPS located at the surface of the ethanol was removed with a spatula and resuspended in H₂0 under constant gentle shaking for 2 h. The solution was dialysed against H₂0 in a dialysis tubing (14 kDa, Sigma) at 4°C and consequently applied 2 times on SPE C18 columns (Bakerbond) as indicated by the manufacturer using a HyperSep-96™ vacuum manifold (Thermo Scientific). The flow-through fraction was collected filtered through 0.45µm syringe filters. The solution was aseptically filled in glass vials and freeze-dried (Virtis Genesis). Dry EPS was stored at -80°C until further usage.

### Example 2

### Bifidobacterium longum 35624 grown in pH controlled liquid fermentation. Capsular EPS was purified from the cell surface with NaOH.

Glycerol stocks of *Bifidobacterium longum* NCIMB 41003 (35624™) were reactivated in RCM (Oxoid) containing 0.05% cysteine and incubated for 48 h at 37°C under anaerobic conditions. The culture was used to inoculate 100 ml of MRS media (Oxoid) containing 0.05% L-cysteine under the same conditions. The resulting culture was used to inoculate 3 1 MRS media (Oxoid) supplemented with additional 3% glucose and 0.05% cysteine (final glucose concentration 5%). A constant temperature of 37°C and a constant pH 6 were held throughout the fermentation by addition of NaOH using a RALF fermentation system (Bioengineering). The culture was harvested after 16 h by centrifugation at 12000 g and 4°C (Sorval). Cells were washed 2 times by resuspension in PBS followed by a centrifugation 12000 g and 4°C (Sorval RC6 plus). After washing, cell associated EPS was extracted by a 5 min incubation of the cells in 1 M NaOH under constant stirring. Cells were removed by centrifugation at 20000g for 30 min and 4°C. The supernatant was neutralised with HCl to pH 7 and filtered through 0.45 µm. Ribonuclease A (1 µg/ml) and Deoxyribonuclease I (5 µg/ml Sigma) were added and incubated for 2 h at room temperature. The solution was added to 3 volumes of ethanol and the precipitating EPS accumulating at the surface of the ethanol was recovered. The EPS was solubilized in distilled water and dialysed against distilled water (14 kDa, Sigma). The solution was filtered through 45 µm syringe filter and applied 2 times on SPE C18 columns (Bakerbond) as indicated by the manufacturer using a HyperSep-96™ vacuum manifold (Thermo Scientific). The flow-through fraction was collected filtered through 0.45 µm syringe filters. The salt free solution as aliquot in sterile glass vials and freeze dried (Virtis Genesis). Dry EPS was stored at -80°C until further usage.

### Example 3

### Bifidobacterium longum NCIMB 41003 (35624™) grown in pH controlled liquid fermentation. Secreted EPS was purified from the fermentation broth by collecting the aggregations.

Glycerol stocks of *Bifidobacterium longum* NCIMB 41003 (35624™) and reactivated in RCM (Oxoid) containing 0.05% cysteine and incubated for 48 h at 37°C under anaerobic conditions. The culture was used to inoculate 100 ml of MRS media (Oxoid) containing 0.05% L-cysteine under the same conditions. The resulting culture was used to inoculate 3 1 MRS media (Oxoid) supplemented with additional 3% glucose and 0.05% cysteine (final glucose concentration 5%). A constant temperature of 37°C and a constant pH 6 were held throughout the fermentation by addition of NaOH using a RALF fermentation system (Bioengineering). The culture was harvested after 16 h by centrifugation at 8000 RPM and 4°C (Sorval). Cells were discarded and residual cells in the supernatant were removed by filtration through a 0.45 µm filter, ribonuclease A (1 µg/ml) and deoxyribonuclease I (5µg/ml Sigma) was added and followed by a 2 h incubation at room temperature. The filtrate was added to 3 volumes of chilled ethanol and gently mixed for 30 seconds. The solution was incubated for 2 h at 4°C and the precipitating EPS accumulating at the surface of the ethanol solution was removed and resuspended in PBS. The precipitation in ethanol and the resuspension in PBS was repeated 2 times. The final resuspension of the EPS was performed in distilled water. The solution was dialysed against distilled H₂O to remove residual ethanol. Salt free EPS solution was aliquoted into sterile glass vials and freeze-dried (Virtis Genesis). Dry EPS was stored at -80°C until further usage.

The chemical structure of the *Bifidobacterium longum* NCIMB 41003 (35624™) secreted exopolysaccharide was determined using a combination of chromatography for constituent analysis, gas chromatography for linkage determination, mass spectrometry for sequencing and various NMR techniques including ¹H, ¹³C HSQC, HSQC-TOCSY and ROESY NMR. The polysaccharide contained a branched hexasaccharide repeating unit with two galactoses, two glucoses, galacturonic acid and the unusual sugar 6-deoxytalose.

### Initial characterization

Size exclusion chromatography was performed with a PL aquagel-OH MIXED-H 8 µm, column (300 x 7.5 mm, Agilent, Waldbronn, Germany) at flow rate of 0.5 mL/min at room temperature with 25 mM ammonium acetate (pH 8.5) with a refractive index detector. Dextran standards with nominal mass (M_{w}) of 25, 150 and 1100 kDa were used for comparison.

AIEX on EconoPac-Q cartridge (Bio-Rad, Vienna, Austria), EPS applied in 50 mm ammonium acetate, eluted with NaCl gradient. Fractions were analyzed for carbohydrate by the orcinol-sulfuric acid method.

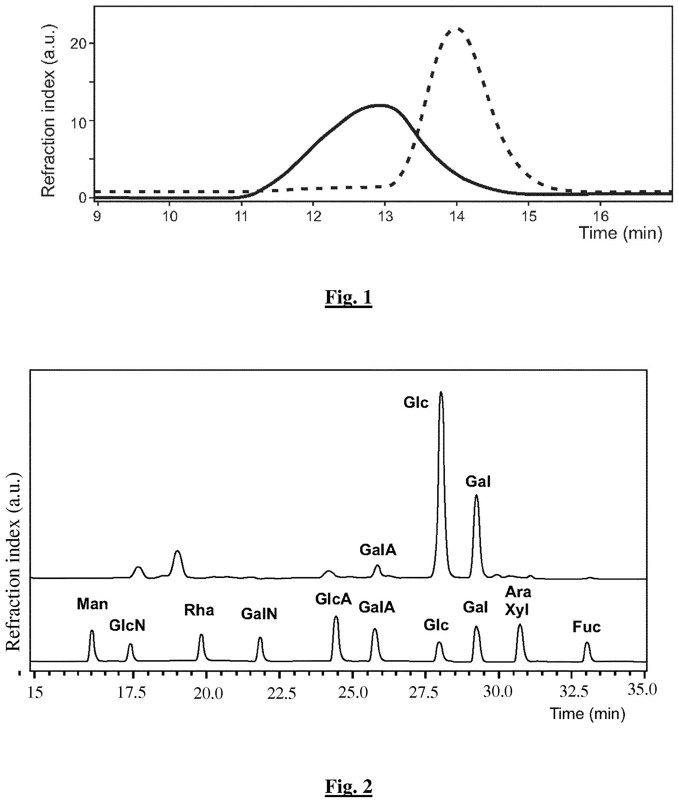

The *Bifidobacterium longum* NCIMB 41003 (35624™) exopolysaccharide dissolved slowly and gave a viscous solution. Comparison with dextran standards by high performance size exclusion chromatography (SEC) pointed at an average mass much higher than 1 MDa (M_{w}) (Fig. 1). Monosaccharide analysis of SEC fractions gave the same composition for all fractions of the broad peak. Anion exchange of the EPS revealed it as being negatively charged.

### Monosaccharide composition

EPS was hydrolyzed with 4 M trifluoro acetic acid at 100°C for 4 h. The monosaccharides were derivatized with anthranilic acid and analyzed by reversed phase HPLC using a 5 µm Kinetex C18 core-shell column (Phenomenex, Torrens, CA) and an acetonitrile gradient in either 0.2 % 1-butylamin, 0.5 % phosphoric acid, 1 % tetrahydrofuran [Anumula K.R. 1994 Anal Biochem. 220, 275-283] or in 0.3 % formic acid buffered to pH 3.0 with ammonia in order to allow subsequent mass spectrometric verification of peaks.

High-performance liquid chromatography of monosaccharides as 1-phenyl-3-methyl-5-pyrazolone (PMP) derivatives was performed on a Hypersil ODS column as described [Honda S1, Akao E, Suzuki S, Okuda M, Kakehi K, Nakamura J. 1989 Anal Biochem. 180 351-357 // Stepan H, Staudacher E. 2011 Anal Biochem. 418, 24-]

Gas chromatographic analysis of alditol acetates was performed as described [Saamanen, A. & Tammi, M. (1988) Glycoconjugate J. 5, 235-243.] with a 60 m OPTIMA® 1 MS Accent column with 0.25 mm inner diameter and 0.25 µm film thickness (Macherey-Nagel, Düren, Germany) with mass spectrometric detection on a GC System 7820A with coupled to a MSD5975 (both Agilent, Waldbronn, Germany).

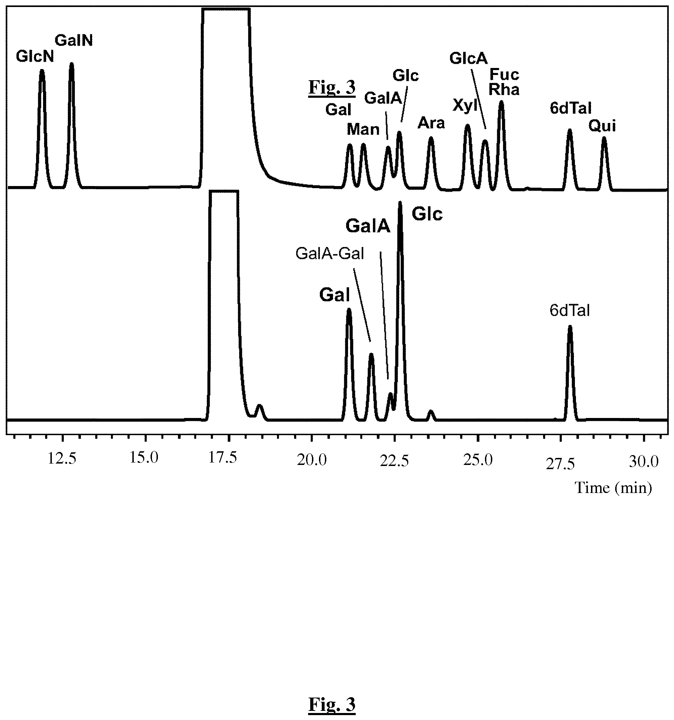

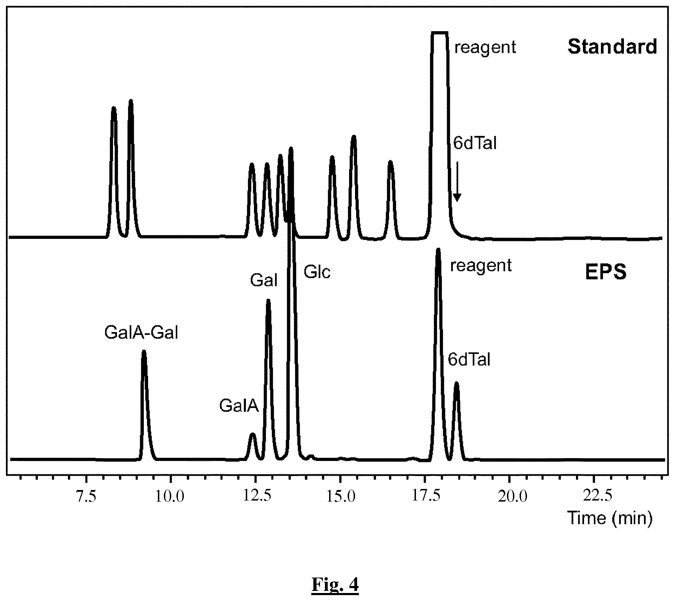

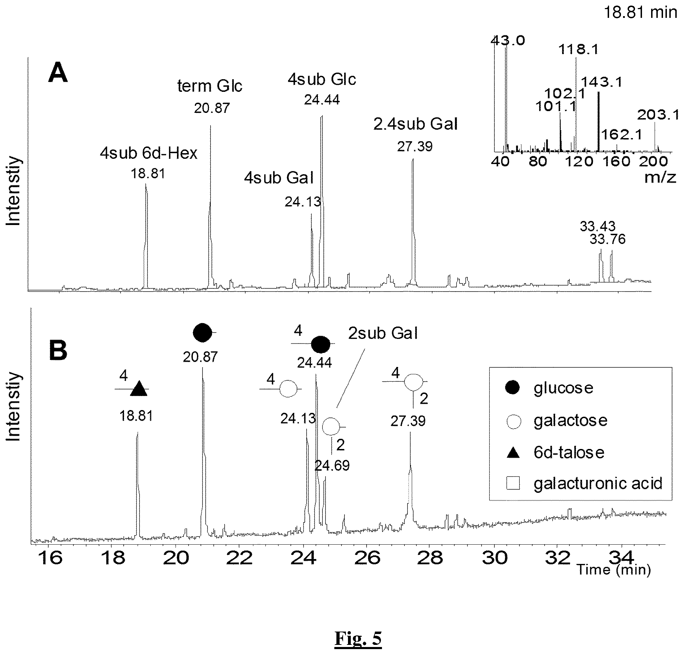

Analysis of the EPS hydrolysates was accomplished by HPLC of sugars derivatized with either 1-phenyl-3-methyl-5-pyrazolone (PMP) or anthranilic acid using at first the standard solvent systems. This implied the occurrence of glucose (Glc), galactose (Gal) and a small amount of galacturonic acid (GalA) (Fig. 2 and 3). Anthranilic acid derivatives were then analyzed with a volatile buffer allowing mass spectrometry of isolated peaks, which revealed one unknown peak and another one as a deoxy-hexose that did not co-elute with fucose, rhamnose or quinovose (Fig. 4). Gas chromatography-mass spectrometry (GC-MS) of partially methylated alditol acetates as well as ¹H-nuclear magnetic resonance (NMR) identified it as 6-deoxy-hexose (Fig. 5). With NMR data pointing at 6-deoxy-talose (6dTal), this rare sugar was synthetized from L-fucose by epimerization at C-2. The deoxy-hexose indeed co-eluted with 6dTal (Figs. 3 and 4). Adding the area of the aldobiuronic acid to Gal and GalA, the molar ratio of the constituent sugars Glc, Gal, GalA and a 6dTal approximated 2: 2 : 1 : 1, respectively.

Mild acid treatment (0.25 M TFA for 4 h at 80°) gave a range of oligosaccharides as seen by porour graphitic-liquid chromatography-electrospray ionization-mass spectrometry (PGC-LC-ESI-MS). Many isobaric fragments indicated lack of a preferred cleavage site (Fig. 6). The smallest of the major fragments was the tetrasaccharide os211 consisting of 2 Hex, 1 GalA and 1 dHex residue. BD₄-reduction and ESI-MS/MS identified the reducing end as hexose. Os211 gave only one isomer and this allowed the preparative isolation of os211 by HILIC HPLC for NMR analysis. The os311 and os411 fragments, when analyzed by PGC-LC-ESI-MS/MS turned out to contain at least 3 isomers with hexose and 1 with deoxyhexose at the reducing end. Reducing end dHex implies this sugar to be part of the main chain rather than a substituent to it. These isomers were isolated by preparative PGC for MS/MS in permethylated form.

### Linkage analysis

Linkage analysis by gas liquid chromatography with electron impact-mass spectrometry (GLC-MS) was conducted after hydrolysis of EPS with the mildest conditions that provided solubility in dimethyl sulfoxide, *i.e.* 50 mM trifluoroacetic acid at 80°C for 4 h. Permethylation was achieved with sodium hydroxide / methyl iodide [Ciucanu, I. & Kerek, F. (1984) Carbohydr: Res. 131, 209-217.] followed by hydrolysis, reduction with sodium borodeuteride and acetylation. The resulting partially methylated alditol acetates were separated on a 60 m OPTIMA® 1 MS Accent column with 0.25 mm inner diameter and 0.25 µm film thickness (Macherey-Nagel, Düren, Germany). Retention time standards were obtained by derivatizing glucose or galactose with sub-stoichiometric amounts of methyl iodide, which worked well for tetra- and tri-methyl ethers.

Permethylation of the intact polysaccharide failed due its insolubility in DMSO. Results were obtained upon gentle hydrolysis with 50 mM TFA (4 h 80°). Peaks for a 4-substituted 6-desoxy-hexopyranose (or a 5-substituted furanose), terminal Glc, 4-substituted Gal and 2,4-disubstituted Gal were obtained in a ratio of about 0.8 : 1 : 0.6 : 1 : 1 (Fig. 5). The uronic acid cannot be seen by this approach. Taking into account the incomplete hydrolysis of the uronic acid's glycosidic linkage as its acid function is regained during the hydrolysis step and the higher volatility of dHex, this insinuates that each linkage variants occurs just once in the repeating unit.

Permethylation linkage analysis of fragment os211 revealed terminal Glc, 4-substituted 6-deoxy-hexose and a 4-substituted Gal at the reducing end. The occurrence of two singly substituted and one terminal residue necessitates a linear topology of os211. As the occurrence of aldobiuronic acid necessitates a HexA-Hex sequence, the only topology of os211 consistent with the hitherto findings is Glc-d6Tal-GalA-Gal.

### MALDI-TOF/TOF MS of permethylated fragments

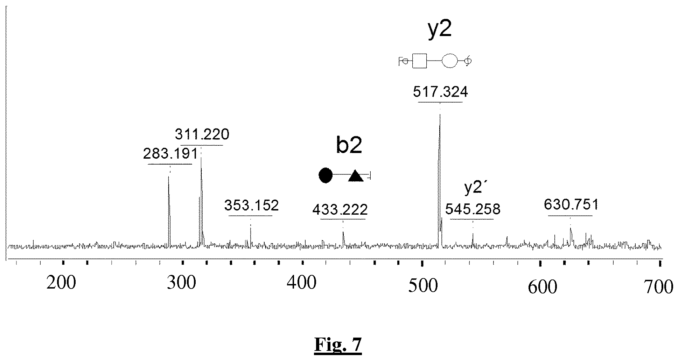

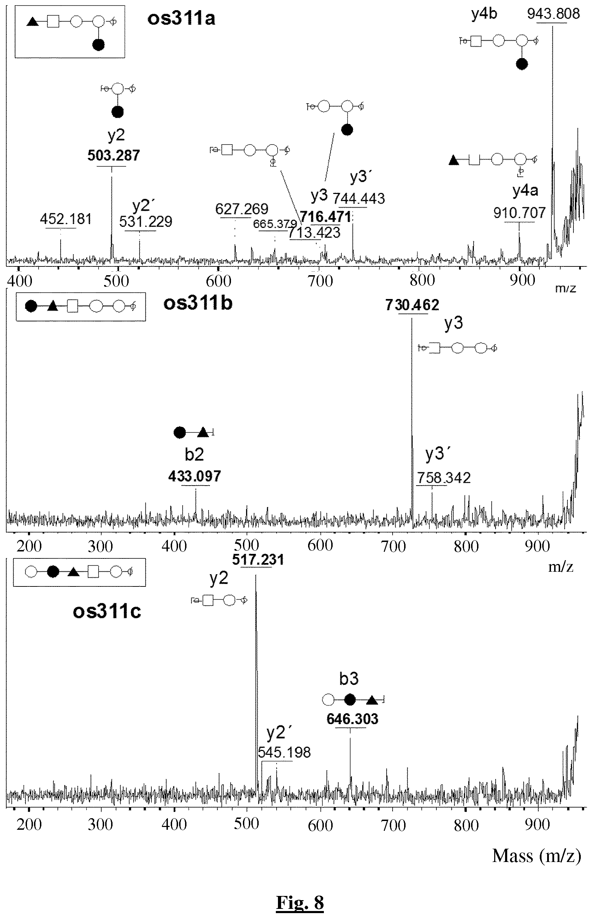

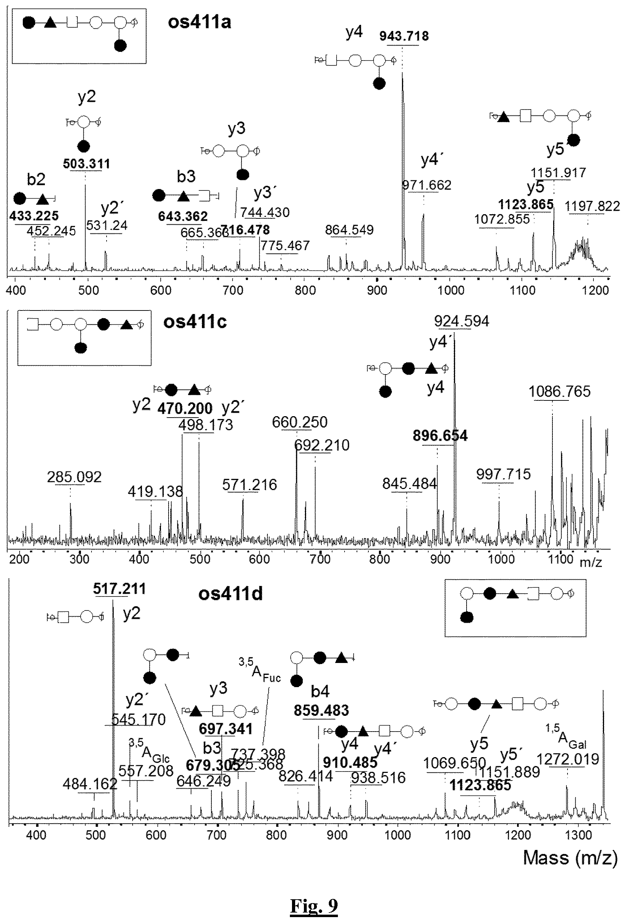

The chromatographically separated, reduced and perdeuteromethylated EPS fragments were analyzed by MS/MS on a MALDI-TOF/TOF instrument. The single os211 fragment gave one y and one b fragment (Fig. 7) that underpinned the Glc-dHex-GalA-Gal topology. In the spectra of os311a and os411a, y-fragments with two and three hexoses and with three hexoses plus uronic acid were found (Figs. 8 and 9). Necessarily, these y-fragments must have harbored the branching Gal-residue bearing the unsubstituted Glc residue. The topology GalA-(Glc-)Gal-Gal, however, could not lead to a fragment of m/z 503.3, which bears only one fragmentation char. In contrast, the MS/MS spectra for os311a and os411a can be reconciled with the fragment structure GalA-Gal-(Glc-)Gal. Os311a did not show a b-fragment from the non-reducing terminus but os411 presented the disaccharide Hex-dHex (= Glc-dTal). Thus, os311a had the sequence d6Tal-GalA-Gal-(Glc-)Gal and os411a Glc-d6Tal-GalA-Gal-(Glc-)Gal, which is the hexasaccharide supposed to constitute the repeating unit of bif624 EPS.

### Absolute configuration of hexoses

Monosaccharides were released by hydrolysis with 4 M TFA for 4 h at 100 °C and reacted with L-cystein methyl ester (Hara S., Okabe H., Mihashi K. (1987), Chem. Pharm. Bull. 35, 501-506 wie schon oben). The dried sample was then derivatized with N-methyl-N-trimethylsilytrifluoroacetamide containing 1% trimethylchlorosilane. The sugars immediately analyzed on an Optima 1MS Accent analytical column (Macherey-Nagel, Germany, 60 m×0.25 mm i.d., 0.25 µm film thickness, 100 % dimethylpolysiloxane stationary phase) and detected with a 7200 GC-QTOFMS system (Agilent, Waldbronn, Germany) (Chu D.B., Troyer C., Mairinger T., Ortmayr K., Neubauer S., Koellensperger G., Hann S. (2015) Anal. Bioanal. Chem. 407, 2865-2875). Notably, compounds were chemically ionized using methane to give molecular ions such that peaks arising from hexoses, deoxyhexoses and uronic acids could be discriminated. The dominating ion for hexoses was the penta-trimethylsilyl derivative of m/z 658.2931. The non-optimized temperature program started with a 1 min hold at 150°C, a 5°C/min ramp to 270°C, a 1 min hold and then a fast ramp to 310°C.

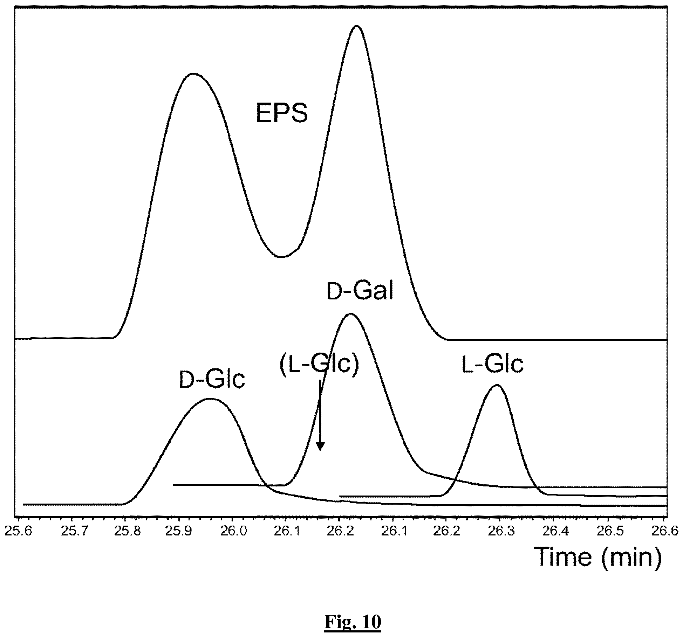

The D/L configuration of galactose and glucose were determined by gas chromatography-mass spectrometry (GC-MS) of trimethylsilylated derivatives of the sugars with L-cystein methylesther (Hara S., Okabe H., Mihashi K. (1987) Chem. Pharm. Bull. 35, 501-506). In order to exclude ambiguities arising from the uronic acid and the deoxy-sugar, for which no enantiomeric standards are available, detection was performed by chemical ionization mass spectrometry. The result revealed the presence of D-glucose and D-galactose (Fig. 10).

### NMR analysis of Bifidobacterium longum NCIMB 41003 (35624™) exopolysaccharide

NMR spectra of the polysaccharide and tetrasaccharide sample were obtained for solutions in 99.9% D₂O (0.6 mL) at 338 K on a Bruker Avance III 600 instrument (600.2 MHz for ¹H, 150.9 MHz for ¹³C) equipped with a BBFO broad-band inverse probe head and z-gradients using standard Bruker NMR software TopSpin 3.0. ¹H spectra were referenced using DSS as standard (δ = 0); ¹³C spectra were referenced using 1,4-dioxane as external standard (δ = 67.40). In general, sweep widths of 5000-6000 Hz for ¹H and 32000-36000 Hz for ¹³C were used. ¹H, ¹H-COSY experiment were measured using the pulse program cosygpqf. ¹H, ¹³C-HSQC spectra were obtained using the pulse program hsqcedetgp with 1024 x 64 k data points and 600 scans per t₁-increment. The *J* value for the HSQC and HMBC experiments was 145 Hz for one-bond couplings. Double-quantum filtered ¹H,¹³C-HMBC experiments were recorded using the pulse program hmbcgpndqf with 4096 x 64 data points, 520 scans per t₁-increment, and values for long range *J*_{X,H} coupling of 11 and 5 Hz, respectively. The HSQC-TOCSY experiment was performed using the pulse program hsqcdietgpsisp with 1024 x 128 data points and 260 scans per t₁-increment. The TOCSY experiment was recorded using the program mlevphpp with 1024 x 256 k data points and a mixing time of 120 ms. ROESY spectra were measured using the pulse program roesyphpp.2, 2048 x 256 data points and a ROESY-spinlock pulse of 0.5 sec. Data were processed using a squared sine-bell function and zero filling one time in the f1 dimension.

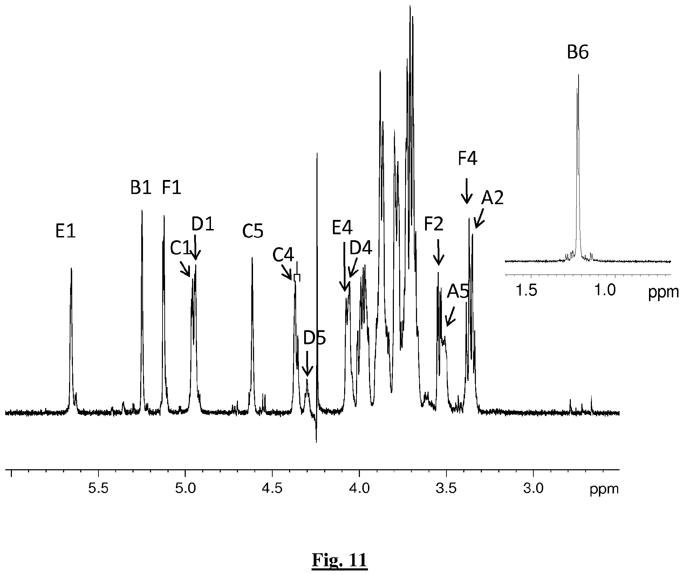

The 600 MHz ¹H-NMR spectra of the acid-treated exopolysaccharide sample were recorded in D₂O at 297 K and 338 K, respectively. Since the latter condition led to better resolved signals, the ensuing ¹³C NMR as well as COSY, TOCSY, HSQC-TOCSY, HMBC and ROESY spectra were recorded at 338 K throughout. The proton spectrum (Fig. 11) revealed *inter alia* six signals of equal intensity attributable to anomeric protons which gave HSQC-correlations to connected carbons in the range of 97-104 ppm. The absence of anomeric carbon signals shifted to lower-field than 104 ppm and of any signals of non-anomeric carbons at a field lower than 82 ppm confirmed that furanose forms were not present (Bock, K.; Pedersen, C. Adv. Carbohydr. Chem. Biochem. 1983 (41) 27-66. // Duus, J. Ø.; Gotfredsen, C. H.; Bock, K. Chem. Rev. 2000 (100) 4589-4614.). Five of the anomeric signals (**B1-F1**) had small homonuclear nuclear coupling constants, while residue **A** displayed a larger coupling constant *J*_{1,2} (∼ 8 Hz). The assignment of the α-anomeric configuration for pyranose residues **B-F** was confirmed by the values of the heteronuclear coupling constant *J*_{C-1,H-1} (observed in an HMBC experiment) that were found in a range of 170-176 Hz. The coupling constant *J*_{C-1,H-1} for residue **A** was consistent the β-anomeric configuration (167.9 Hz) (Bock, K.; Pedersen, C. J. Chem. Soc. Perkin Trans 2, 1974, 293-297.). In the high-field segment of the ¹H-NMR spectrum a methyl group signal being characteristic of a 6-deoxy-pyranose was found at 1.22 ppm.

In the low-field section of the spectrum, two additional, non-anomeric and spin-coupled proton signals were seen (C4, C5). The signal observed at 4.62 ppm (C5) revealed an HMBC connectivity to a carbon signal at 175.3 ppm, thus indicating the presence of a pyranosyluronic acid (C).

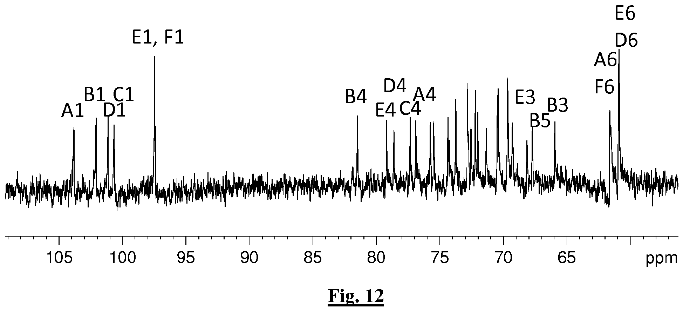

The presence of a hexasaccharide repeat unit was confirmed by analysis of the 150 MHz ¹³C NMR spectrum which contained four signals of anomeric carbons in the region of 100-104 ppm (**A-D**), and a slightly high-field shifted signal of double intensity at 97.5 ppm (**E,F**) (Fig. 12). Pyranose ring carbon signals were present in the range between 65.9 and 81.5 ppm, while methylene carbons (identified via an APT experiment) were shown as two broadened singlets of double intensity at 61.6 and 60.9 ppm, respectively. Additionally, a carbonyl signal originating from the uronic acid moiety was observed at 175.3 ppm, as well as the carbon signal of the methyl group of the 6-deoxy sugar at 16.4 ppm.

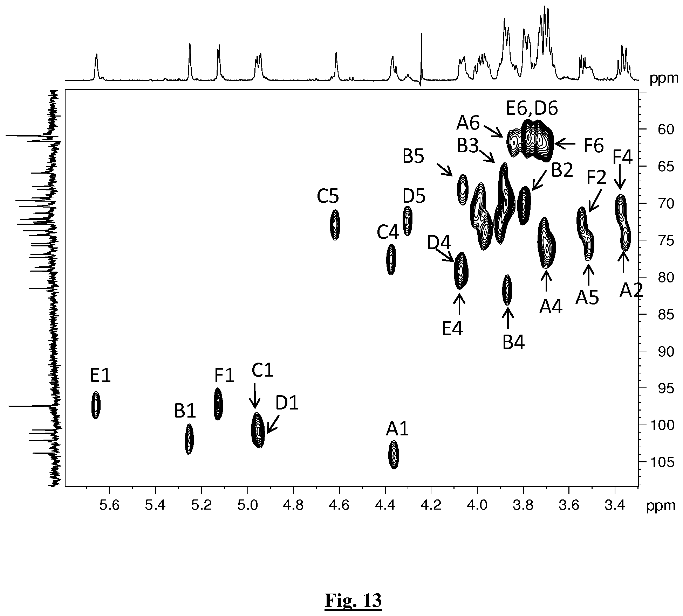

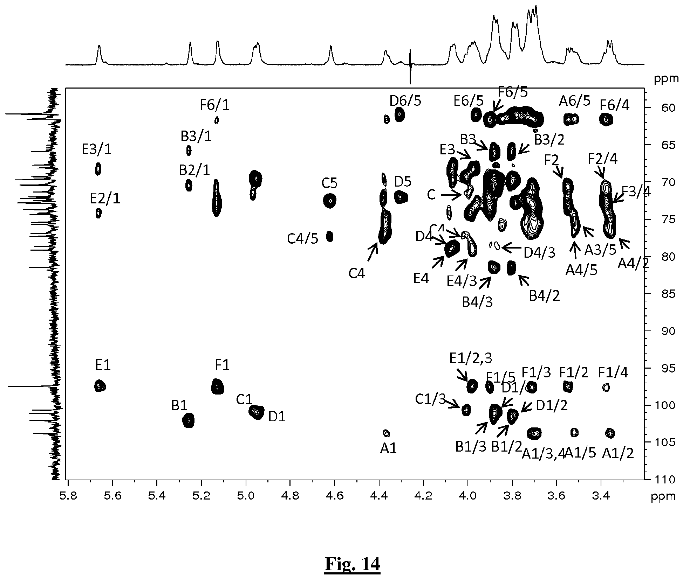

Proton and carbon signals were then assigned using COSY, TOCSY, HSQC, HSQC-TOCSY (Fig. 13, Fig. 14), HMBC and ROESY experiments (Table 1). Glycosylation shifts were seen for units **A-E**, whereas residue **F** occurred as an unsubstituted sugar. Glycosylation sites were identified at position 4 of residues **A-E.** Residue **E** was found to be additionally substituted at carbon 2, based on an HMBC correlation from H-1 of **F** to C-2 of **E.** Furthermore, both anomeric protons of residues **E** and **F** gave inter-residue ROEs in support of a close spatial proximity. The observed substitution pattern was thus in full agreement with the results of the permethylation analysis.

The absolute configuration of constituent sugars was derived from the analysis of the TMS L-cysteine methyl ester derivatives (Hara, S. Chem. Pharm. Bull. 1987 (35) 501-506). In combination with chemical shift information (a) Czerwicka, M.; Forsythe, S. J.; Bychowska, A.; Dziadziuszko, H.; Kunikowska, D.; Stepnowski, P.; Kaczynski. Carbohydr. Res. 2010 (345) 908-913; b) Niedziela, T.; Jachymek, W.; Lukasiewicz, J.; Maciejewska, A.; Andersson, R.; Kenne, L.; Lugowski, C. Glycobiol. 2010 (20) 207-214; c) Perepelov, A. V.; Wang, Q.; Senchenkova, S. N.; Feng, L.; Shashkov, A.S.; Wang, L.; Knirel, Y. A. Carbohydr. Res. 2013 (368) 57-60; d) Stone, J. K.; Heiss, C.; Wang, Z.; Black, I.; Grasso, S. A.; Koppisch, A. T., Azadi, P.; Keim, P.; Tuanyok, A. Carbohydr. Res. 2014 (386) 68-72; e) Katzenellenbogen, E.; Kocharova, N. A.; Toukach, P. V.; Górska, S.; Bogulska, M.; Gamian, A.; Knirel, Y. A. Carbohydr. Res. 2012 (355) 56-62.) and the results of the monosaccharide composition analysis, this led to the assignment of a *D-gluco* configuration for residues **A** and **F** and a *D-galacto* configuration for residues **C**, **D** and **E**, respectively.

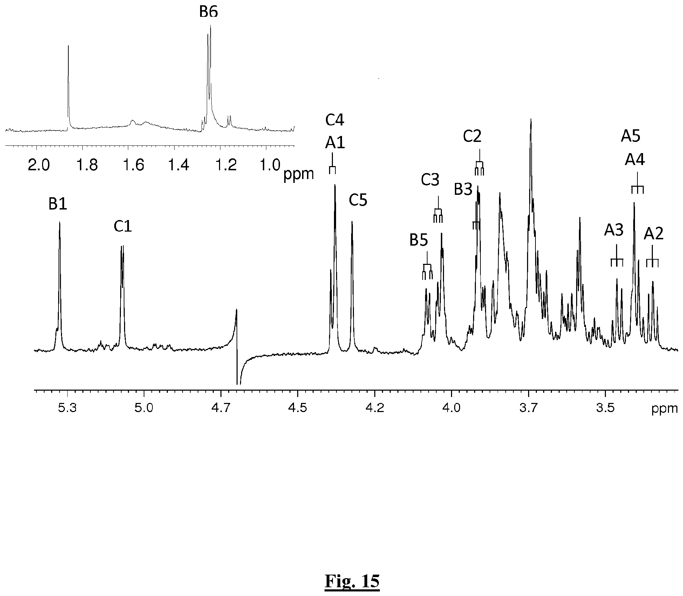

Key assignments were then obtained and confirmed from the tetrasaccharide fragment os211 generated by acid treatment (0.25 M TFA, for 3 h at 80 °C) of the exopolysaccharide followed by borohydride reduction and chromatographic purification. While data of the reduced hexitol component could not be extracted due to severe signal overlap (Fig. 15), the proton signals of three pyranose units **A**, **B**, **C** could be fully assigned based on COSY and TOCSY experiments. This allowed to identify the configuration of the constituent sugars based on homonuclear *J*_{H,H} coupling constants as well as ¹H chemical shift information (Table 1). Residue **A** was assigned as a β-glucopyranosyl residue as seen from the *J*_{2,3} and *J*_{3,4} coupling constants being consistent with a *trans*-diaxial arrangement of the respective protons. Signals arising from residues **B** and **C** were close to those observed for the related units in the polysaccharide chain, whereas H-3 and H-4 signals of unit **A** were shifted to higher field, indicating that hydrolysis had occurred at the residue preceding unit **A.** The small values of the coupling constants *J*_{1,2}, *J*_{3,4} and *J*_{4,5} in conjunction with the large values seen for *J*_{2,3}, identified residue **C** as α-galacturonic acid, whereas residue **B** corresponded to an α-anomeric 6-deoxy-sugar. The coupling constants for the low-field shifted H-5 and H-3 protons of residue **B** revealed small values for *J*_{4,5}, *J*_{3,4} and *J*_{2,3}, respectively, which are compatible with the configuration of a 6-deoxy-talose or a 6-deoxy-gulose.

The absolute configuration of the 6-deoxy-sugar was eventually based on the molar rotation values calculated for the polysaccharide. Published optical rotation values of methyl glycosides were used for the calculation (a) Mori, M.; Tejima, S.; Niwa, T. Chem. Pharm. Bull. 1986 (34) 4037-4044; // b) Bitzer, J.; Zeeck, A.; Eur. J. Org. Chem. 2006, 3663-3666). For a hexasaccharide repeat unit containing an α- and β-D-glucopyranosyl unit, two α-D-galactopyranosyl units, one α-D-galactopyranosyluronic residue and a 6-deoxy-α-talopyranose unit, the calculated molar rotation [M_{D}] gives 1221.5 for 6-deoxy-α-D-talose as constituent sugar and 844.3 for the presence of 6-deoxy-α-L-talose, which would correspond to optical rotation values of +126 and +87, respectively. The measured optical rotation value [α]_{D}²⁰+84.7 (*c* 0.96, H₂O) was in full agreement with the presence of an 6d-L-talopyranose. This assignment was further supported by characteristic chemical shift differences observed for anomeric carbons when engaged in linkages between L,D- and D,D-configured sugars (Lipkind, G. M.; Shashkov, A. S.; Knirel, Y. A.; Vinogradov, E. V.; Kochetkov, N. K. Carbohydr. Res. 1988 (175) 59-75. // Jansson, P.-E.; Kenne, L.; Widmalm, G. Carbohydr. Res. 1989 (188) 169-191.) The presence of rhamnose, fucose and 6-deoxy-quinovose was excluded based on the results of the HPLC monosaccharide analysis, while the ¹³C NMR chemical shifts observed for residue **B** were not compatible with the presence of a 6-deoxy-gulose, 6-deoxy-altrose and 6-deoxy-allose (Bock 1983, Lipkind 1988). The NMR data of the 6-deoxy-pyranose showed a diagnostic high-field shifted ¹³C NMR signal as seen for C-3 of talose¹ and were also in good agreement with published NMR features of a disaccharide fragment β-D-Glc*p*-(1→4)-α-L-6dTalp occurring in *Burkholderia caribensis* strain MWAP71 (Vanhaverbeke, C.; Heyraud, A.; Achouak, W.; Heulin, T. Carbohydr. Res. 2001 (334) 127-133.). The identity of the deoxy-sugar was eventually proven using a synthetic sample of L-6dTal.

HMBC data (two experiments with values of *J*_{CH}-coupling constants of 5 and 11 Hz, respectively, were performed) confirmed the assignment of spin systems with characteristic intraresidue correlations between the anomeric protons of residues **C** and **F** to carbon 3, and anomeric protons of residues **A**, **B** and **E** to carbon 5, respectively. In addition the following interresidue connectivities were observed: H-1 of **A** and C-4 of **B**, H-1 of **B and** C-4 of C, H-4 of **E** and C-1 of **D**, H-1 of **F** and C-2 of **E.**

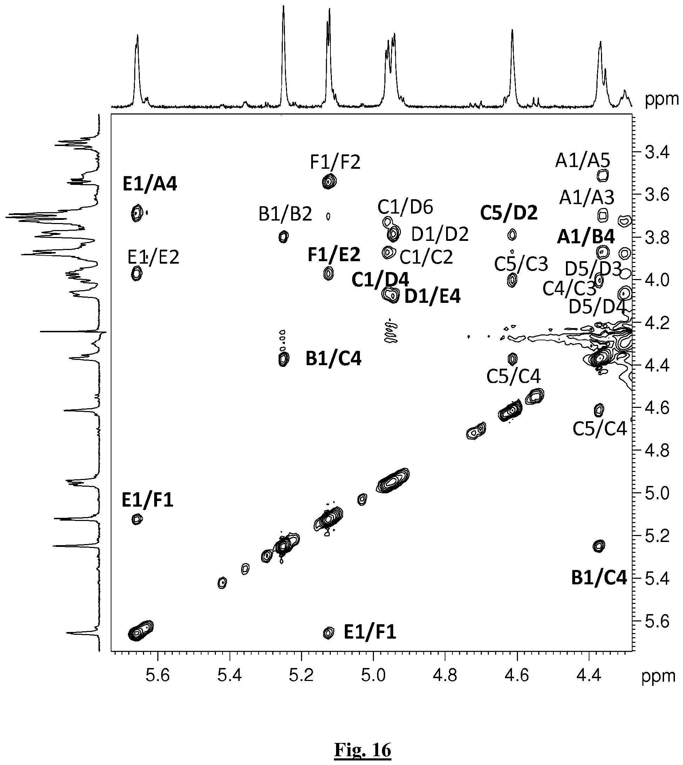

The final assignment of the sequence was based on ROESY intra- and interresidue correlations (Fig. 16).

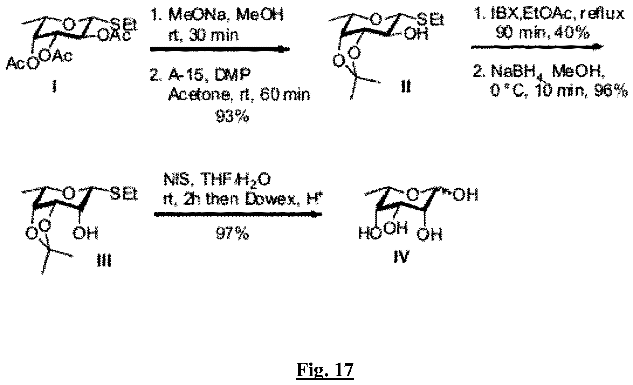

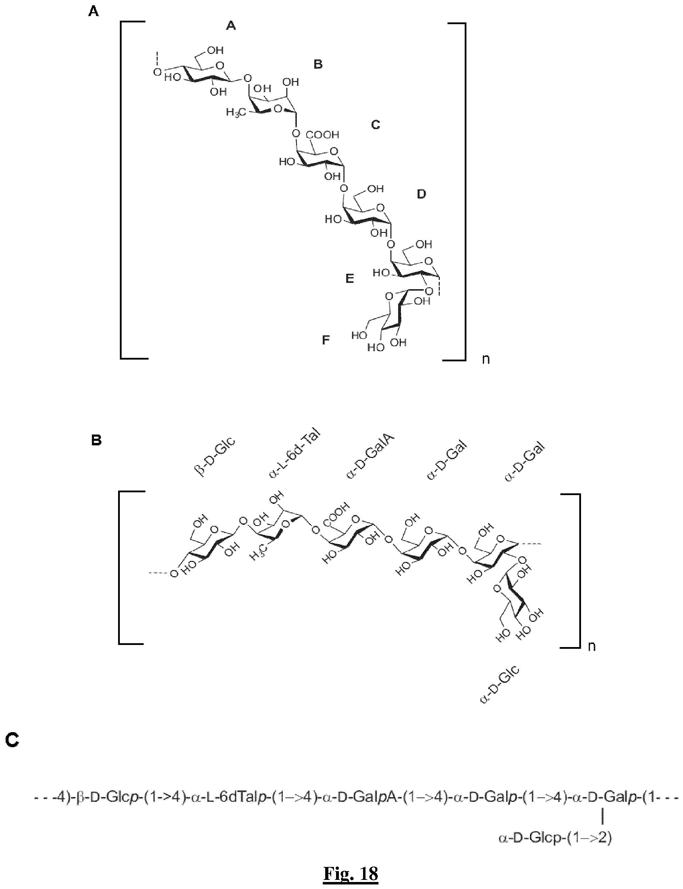

6-deoxy-L-talose was synthesized as follows. Ethylthio β-L-fucopyranoside I was prepared according to literature and was converted into the 3,4-O-isopropylidene derivative II in 93% yield (Veeneman, G. H.; van Leeuwen, S. H.; Zuurmond, H.; van Boom, J. H. J. Carbohydr. Chem. 1990 (9) 783-796. // Smid, P.; de Reuiter, G. A.; van der Marel, G. A.; Rombouts, F. M.; van Boom, J. H. J. Carbohydr. Chem. 1991 (10) 833-849.). While epimerization using Mitsunobu conditions or triflate-displacement failed, oxidation by 2-iodoxybenzoic acid (IBX) followed by selelective reduction of the intermediate 2-ulose proceeded smoothly and afforded the talo-product III (Scheme S1). Deprotection via hydrolysis of the thioglycoside and cleavage of the 3,4-O-acetonide afforded the 6-deoxy-L-talose (IV). The synthesis strategy is represented graphically in Fig. 17.
Based on the combined evidence of sugar analysis, methylation data and NMR-data a structure is proposed for the exopolysaccharide derived from *Bifidobacterium longum* NCIMB 41003 (35624™) as shown in Fig. 18.

**Table 1.**

| ¹H and ¹³C NMR chemical shifts (δ, ppm) of the exopolysaccharide (recorded at 338 K) and the tetrasaccharide os211 (recorded at 300 K) from *Bifidobacterium longum* NCIMB 41003 (35624™) | | | | | | |
|---|---|---|---|---|---|---|
| Sugar residue | H-1 C-1 | H-2 C-2 | H-3 C-3 | H-4 C-4 | H-5 C-5 | H-6 (6a,6b) C-6 |
| →4)-β-D-Glc*p*-(1→ | 4.35 | 3.36 | 3.68 | 3.70 | 3.51 | 3.70, 3.84 |
| **A** | 103.83 | 74.36^{a} | 75.46^{b} | 76.90 | 75.76^{b} | 61.62 |
| | | | | | | |
| →4)-α-L-6-deoxy-Tal*p-*(1→ | 5.25 | 3.80 | 3.88 | 3.86 | 4.06 | 1.22 |
| | 102.10 | 70.49^{c} | 65.93 | 81.50 | 67.74 | 16.38 |
| **B** | | | | | | |
| | | | | | | |
| →4)-α-D-Gal*p*A-(1→ | 4.96 | 3.87 | 4.00 | 4.38 | 4.62 | |
| C | 100.69 | 69.66 | 71.34 | 77.33 | 72.84 | 175.26 |
| | | | | | | |
| →4)-α-D-Gal*p*-(1→ | 4.94 | 3.80 | 3.88 | 4.06 | 4.30 | 3.73 |
| D | 101.16 | 69.31 | 70.43^{c} | 78.63 | 72.22^{d} | 60.89 |
| | | | | | | |
| →2,4)-α-D-Gal*p*-(1→ | 5.66 | 3.97 | 3.98 | 4.08 | 3.96 | 3.78 |
| **E** | 97.47 | 74.22^{a} | 68.16 | 79.19 | 72.02^{d} | 60.89 |
| | | | | | | |
| α-D-Glc*p*-(1→ | 5.13 | 3.54 | 3.71 | 3.38 | 3.90 | 3.71 |
| **F** | 97.47 | 72.54 | 73.74 | 70.80 | 72.80 | 61.62 |

| Tetrasaccharide os211 | H-1 *J* (Hz) | H-2 *J* (Hz) | H-3 *J* (Hz) | H-4 *J* (Hz) | H-5 *J* (Hz) | H-6 (6a,6b) *J* (Hz) |
|---|---|---|---|---|---|---|
| →4)-β-D-Glc*p*-(1→ | 4.385 | 3.35 | 3.46 | 3.39 | 3.40 | 3.71, -3.86 |
| **A** | 7.9 | 8.8 | 9.0 | | | 4.9, 12.5 |
| | | | | | | |
| →4)-α-L-6-deoxy-Tal*p-*(1→ | 5.275 | 3.84 | 3.915 | n.d. | 4.08 | 1.25 |
| | 2.3 | | 3.3 | | 2.4 | 6.6 |
| **B** | | | | | | |
| →4)-α-D-Gal*p*A-(1→hexitol) | 5.07 | 3.90 | 4.04 | 4.38 | 4.32 | - |
| | 3.8 | 10.2 | 2.5 | 1.9 | n.d. | |
| **C** | | | | | | |

| | | | | | | |
|---|---|---|---|---|---|---|
| ^{a,b,c,d} assignments may be reversed | | | | | | |

### Example 4 - Bifidobacterium longum NCIMB 41003 (35624™) exopolysaccharide has immunomodulatory activity when co-incubated with human immune system cells in vitro.

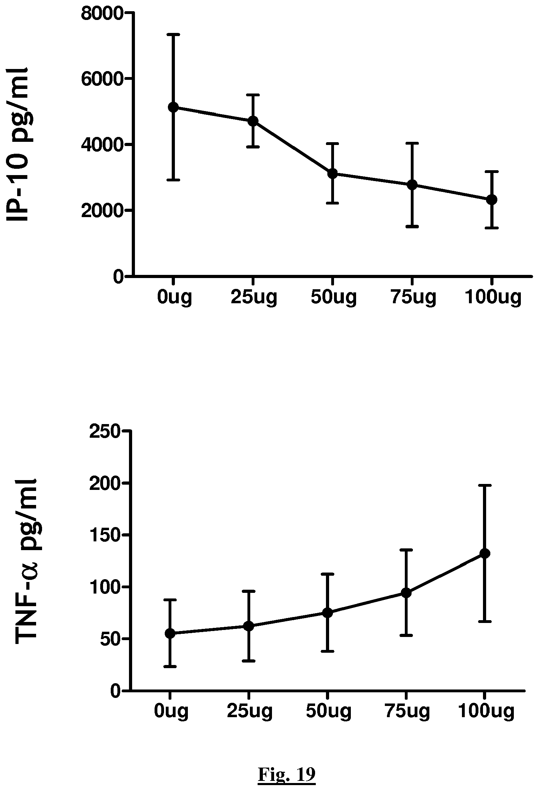

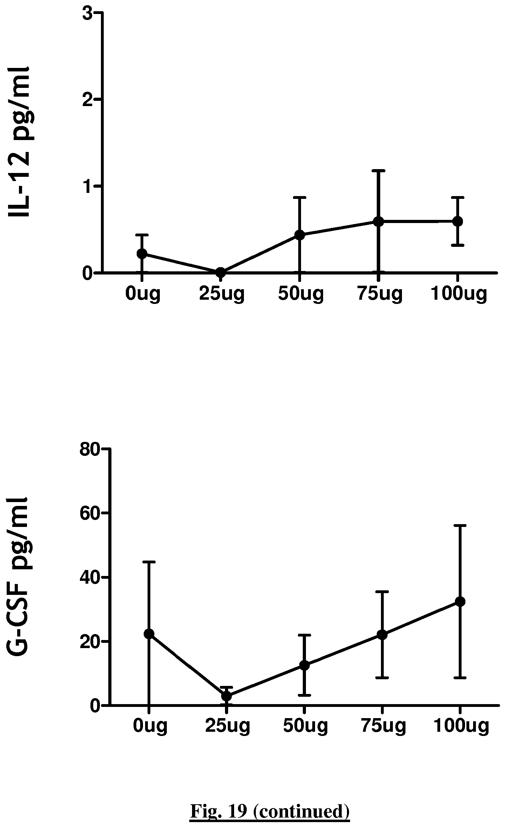

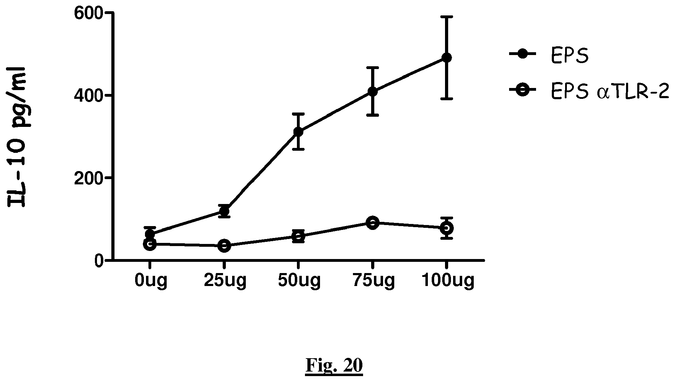

Exopolysaccharide was assayed using monocyte-derived dendritic cells (MDDCs) from healthy human volunteers. MDDCs were generated by culturing human peripheral blood monocytes in GM-CSF and IL-4 for six days. MDDCs were exposed to the isolated polysaccharide for 24 hours and cytokine levels in culture supernatants measured using multiplex bioplex assays. Exopolysaccharide at 25, 50, 75 or 100µg/ml did not induce secretion of the proinflammatory cytokines IP-10, TNF-alpha, IL-12 or G-CSF (Fig. 19). In contrast, exopolysaccharide induced a selective and robust secretion of the anti-inflammatory cytokine IL-10 (Fig. 20). Interestingly, neutralisation of Toll-like receptor 2 (TLR-2) using a monoclonal antibody completely blocked the IL-10 response to the exopolysaccharide (Fig. 20). The secreted and capsular exopolysaccharide displayed the same activity in this in vitro model.

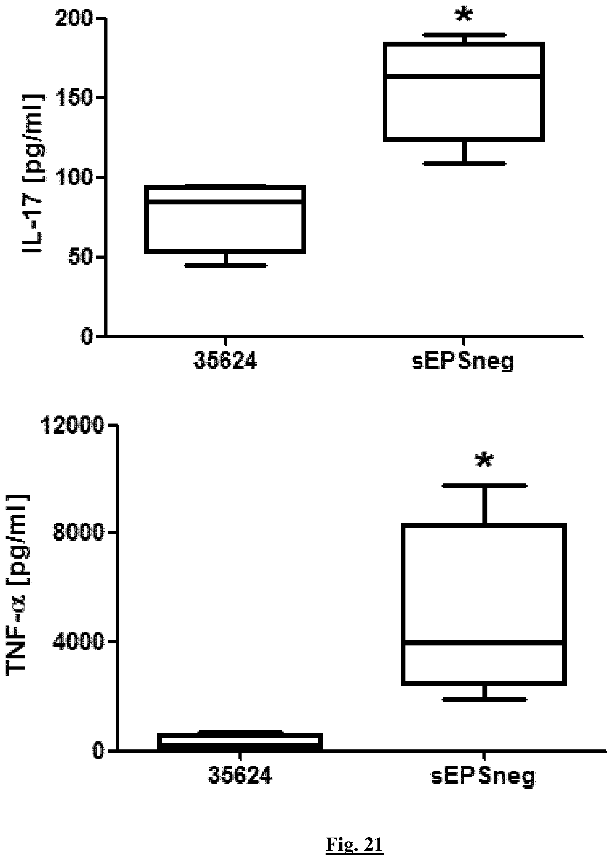

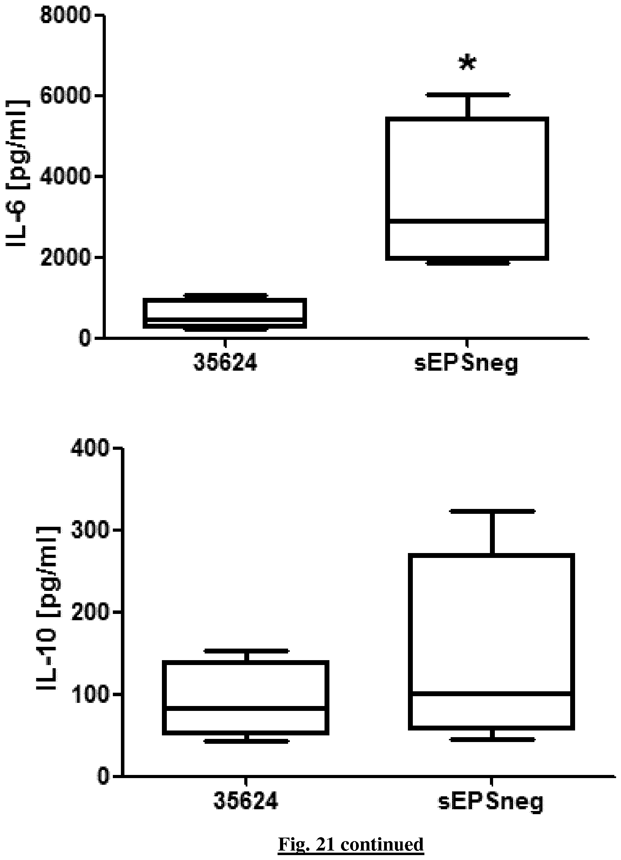

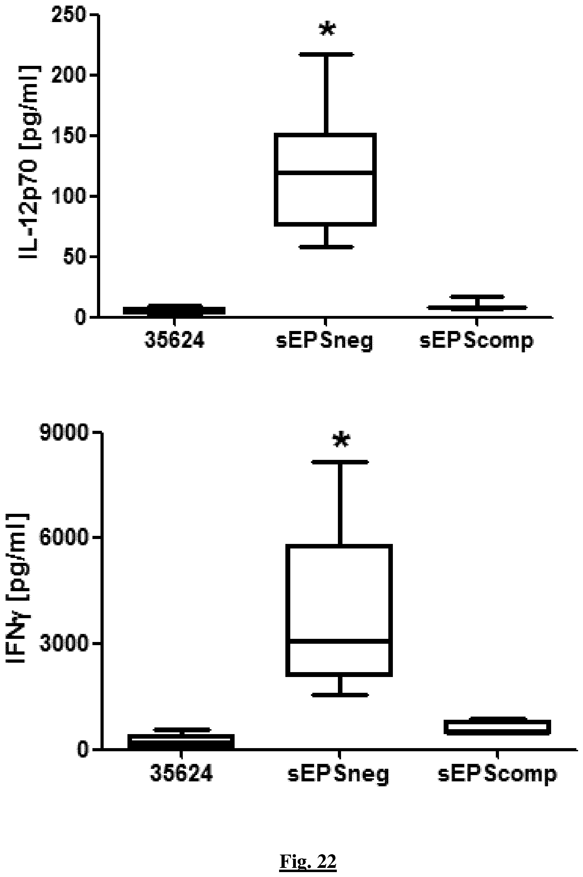

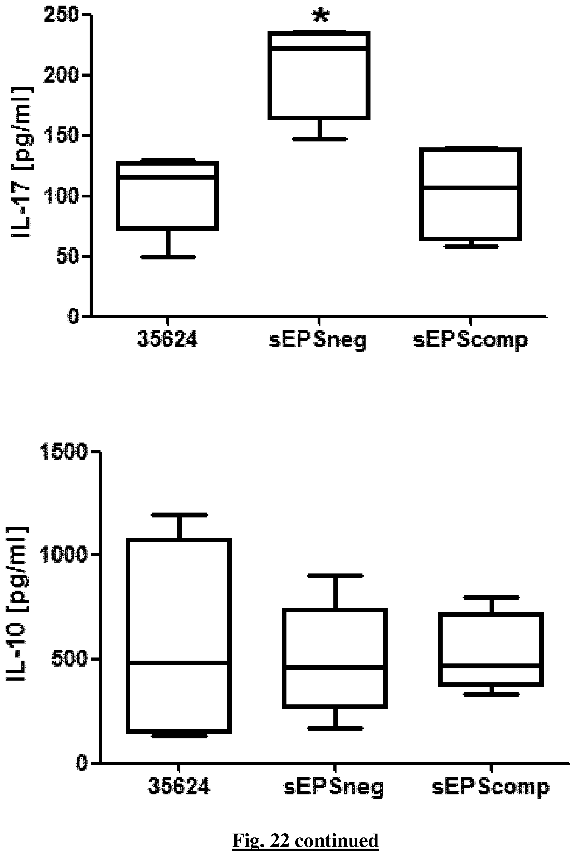

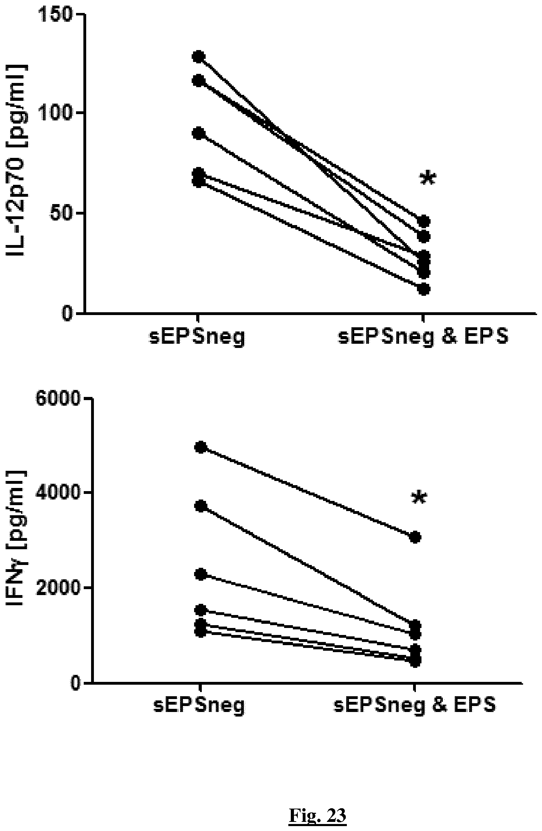

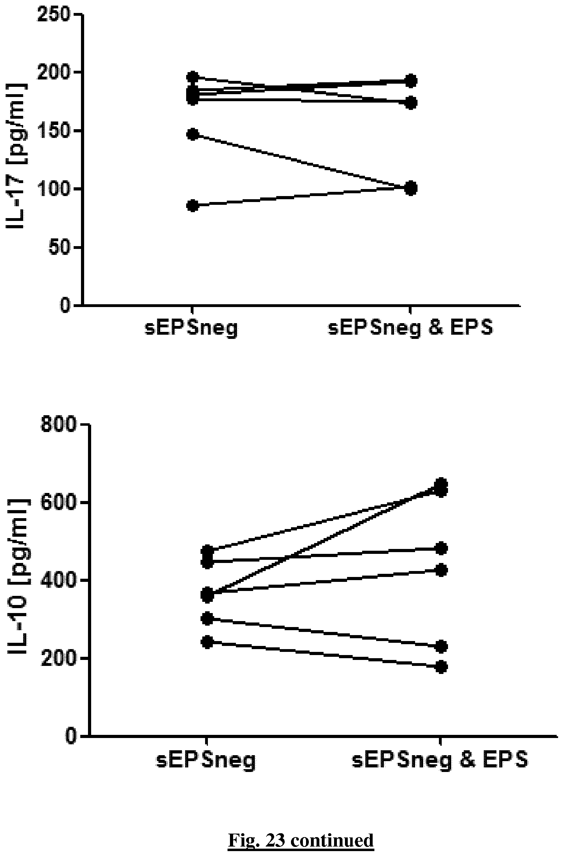

An isogenic exopolysaccharide-negative mutant derivative of *Bifidobacterium longum* NCIMB 41003 (35624™) (termed sEPSneg) was generated and was co-incubated with MDDCs in vitro. Surprisingly, the sEPSneg mutant (which lacks the ability to produce the exopolysaccharide) induced vastly more proinflammatory cytokines, including IL-17, TNF-□ and IL-6 from MDDCs compared to the wild-type *Bifidobacterium longum* NCIMB 41003 (35624™) strain. However, no significant difference in IL-10 secretion was observed (Fig. 21). Human peripheral blood mononuclear cells (PBMCs) were also co-incubated with these bacterial strains and again the sEPSneg mutant induced significantly more IL-12p70, IFN-□ and IL-17 secretion, compared to the wild-type *Bifidobacterium longum* NCIMB 41003 (35624™) strain, with no difference in IL-10 secretion (Fig.22). The exaggerated cytokine response was reversed when exopolysaccharide production was restored in the sEPSneg mutant by genetic complementation (termed sEPScomp, Fig. 22), confirming that enhanced pro-inflammatory cytokine secretion is specifically associated with the lack of exopolysaccharide production. The addition of isolated exopolysaccharide to the PBMC-sEPSneg co-cultures significantly reduced IL-12p70 and IFN-□ secretion in response to the sEPSneg strain, but did not alter IL-17 or IL-10 responses to the sEPSneg strain (Fig. 23).

### Example 5: Bifidobacterium longum NCIMB 41003 (35624™) efficacy in the SCID colitis model is dependent on its ability to express the exopolysaccharide

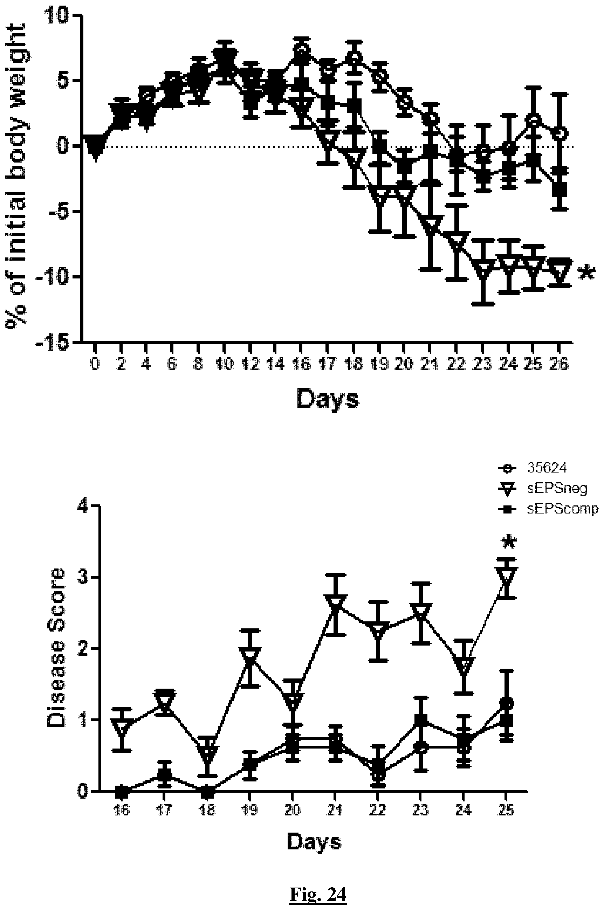

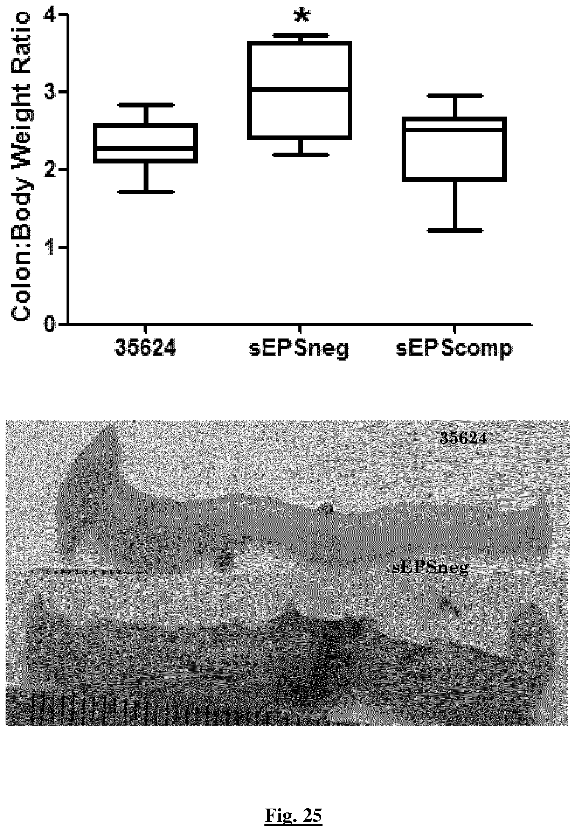

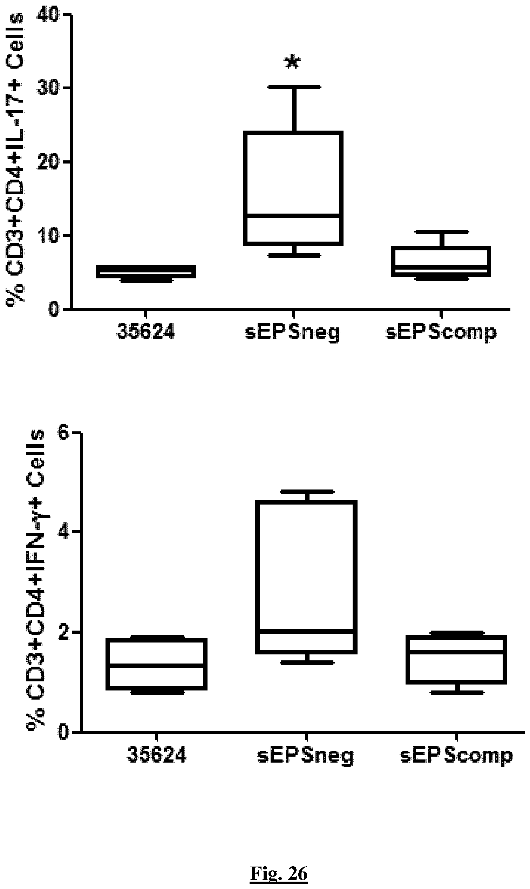

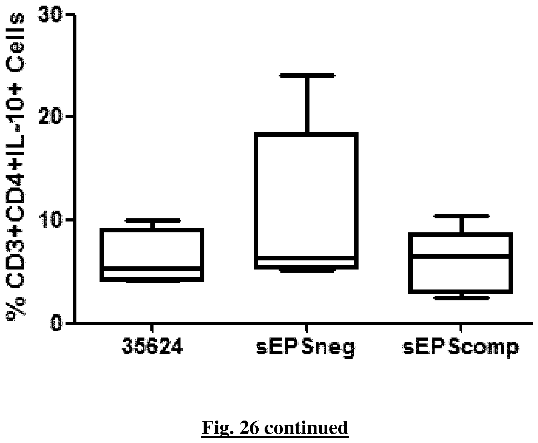

Colitis was induced in SCID mice by adoptively transferring CD4+CD25-CD45RBhi lymphocytes. Mice were administered *Bifidobacterium longum* NCIMB 41003 (35624™) strain, sEPSneg or sEPScomp daily by oral gavage. As previously described, *Bifidobacterium longum* NCIMB 41003 (35624™) strain treatment prevented weight loss and disease symptoms in this model (van der Kleij H, O'Mahony C, Shanahan F, O'Mahony L, Bienenstock J. 2008. Protective effects of Lactobacillus reuteri and Bifidobacterium infantis in murine models for colitis do not involve the vagus nerve. Am J Physiol Regul Integr Comp Physiol 295:R1131-R1137.). However, mice treated with the sEPSneg strain exhibited significant weight loss and severe disease symptoms, while restoration of exopolysaccharide production in the sEPScomp strain promoted a similar response as to the wild-type *Bifidobacterium longum* NCIMB 41003 (35624™) strain (Fig. 24). Following euthanasia, the colon:body weight ratio was significantly higher in animals administered the sEPSneg strain, while macroscopically the colons of these mice appeared severely inflamed with visible necrotic regions, which was not observed when animals had been administered B. longum 35624 (Fig. 25). Within the mesenteric lymph nodes, there were significantly more IL-17+ lymphocytes in animals administered the sEPSneg, with a trend towards increased numbers of IFN-□+ lymphocytes, which was not statistically significant (Fig. 26). No significant difference in IL-10+ lymphocytes was observed.

These data demonstrate that the particular exopolysaccharide produced by *Bifidobacterium longum* NCIMB 41003 (35624™) plays an essential role in dampening pro-inflammatory host responses to the strain and that loss of exopolysaccharide production results in the induction of local TH17 responses IL-10 is an anti-inflammatory cytokine that is an important regulator of several aspects of immune responses (Akdis M, Burgler S, Crameri R, Eiwegger T, Fujita H, et al. Interleukins, from 1 to 37, and interferon-y: receptors, functions, and roles in diseases. J. Allergy Clin Immunol. 2011 Mar;127(3):701-21.). The IL-10 gene maps to a cytokine cluster that includes the genes IL-19, IL-20, IL-24, and IL-26 on chromosome 1q31-32. IL-10 is produced mainly by monocytes, T cells (mainly Tr1 cells), B cells, NK cells, macrophages, and dendritic cells. IL-10 is secreted as a homodimer that consists of 2 subunits, each of 178 amino acids with a molecular weight of approximately 18 kDa. IL-10 directly affects antigen-presenting cell functions by down-regulating the expression of MHC class II and costimulatory molecules on the surface of macrophages and monocytes. IL-10 inhibits the expression of many proinflammatory cytokines, chemokines, and chemokine receptors and mediates allergen tolerance in allergen-specific immunotherapy and after exposure to high doses of allergen. In addition to these indirect effects, IL-10 directly affects T-cell activation by suppressing CD28, CD2, and signaling of the inducible T-cell costimulator via the tyrosine phosphatase SHP-1. In contrast with its inhibitory effects on T cells, IL-10 promotes survival, proliferation, and differentiation of human B cells and increases the production of IgG4. Several mouse models demonstrate the importance of IL-10 in regulation of the inflammatory response. IL-10 knockout mice develop normal lymphocyte and antibody responses but have reduced growth, are anemic, and spontaneously develop chronic colitis.

TLR-2 is a member of the toll-like receptor family, which recognizes pathogen-associated molecular patterns (PAMPs) that are expressed on infectious agents, and mediate the production of cytokines necessary for the development of effective immunity. TLR-2 has been previously shown to respond to lipid-containing PAMPs such as lipoteichoic acid and di- and tri-acylated cysteine-containing lipopeptides. IL-10 secretion by human MDDCs in response to *Bifidobacterium longum* NCIMB 41003 (35624™) was previously shown to be TLR-2 dependent (Konieczna P, Groeger D, Ziegler M, Frei R, Ferstl R, Shanahan F, Quigley EM, Kiely B, Akdis CA, O'Mahony L). Bifidobacterium longum NCIMB 41003 (35624™) administration induces Foxp3 T regulatory cells in human peripheral blood: potential role for myeloid and plasmacytoid dendritic cells. Gut. 2012 Mar;61(3):354-66).

TH17 cells are a subset of CD4+ T helper cells that mediate protective immunity to extracellular bacterial and fungal pathogens, predominantly at epithelial surfaces (Korn T, Bettelli E, Oukka M, Kuchroo VK. 2009. IL-17 and Th17 Cells. Annu Rev Immunol 27:485-517.). Polarization of naive T cells into TH17 cells occurs following T-cell antigen receptor recognition of an MHC class II-bound peptide in the presence of cytokines including TGF-β1, IL-6 or IL-1□. While TH17 cells are required for protective immunity, these cells massively infiltrate the inflamed intestine of inflammatory bowel disease patients, where they produce IL-17 and other cytokines, triggering and amplifying the inflammatory process (Gálvez J. 2014. Role of Th17 Cells in the Pathogenesis of Human IBD. ISRN Inflammation 2014:1-14.). Our data suggests that the *Bifidobacterium longum* NCIMB 41003 (35624™) strain-associated exopolysaccharide prevents the induction of a TH17 response to this bacterium.
In summary, this experiment demonstrates that the bacterial strain, as a mechanism for providing the EPS to the site of action, dampens the pro-inflammatory response through cytokine cascades and reduces the symptoms and severity of colitis. The strain minus the EPS does not have this protective effect, while complementation of EPS expression restores the beneficial effects of the strain. The suppression of TH17 responses and the induction of IL-10 would also be expected to have anti-inflammatory activity when applied to other disease models, including joints, skin and the airways.

### Example 6 - Bifidobacterium longum NCIMB 41003 (35624™) and its' exopolysaccharide has anti-inflammatory activity in the lung when administered intranasally.

Asthma is a chronic and complex inflammatory disease of the airways characterized by airflow obstruction, bronchial hyper-responsiveness and airway inflammation. It is the most common chronic illness of childhood, with up to 20% of children affected in some Western countries. The incidence as well as the number of hospital admissions attributable to asthma continues to rise in both adults and children. The importance of airway inflammation in the disease process has been investigated, and revealed that the asthmatic tissue is characterized by the accumulation of a large number of inflammatory cells (e.g. eosinophils, neutrophils, basophils, mast cells), increased mucus production, epithelial shedding and hypertrophy mucus, smooth muscle cell hypertrophy and submucosal mucus glands hyperplasia/metaplasia and fibrosis. Notably chronic inflammation of the asthmatic lung leads to structural changes, that in turn exacerbate the hyperresponsiveness observed in this disease. While these findings have provided the rationale for the development of multiple therapeutic agents that interfere with specific inflammatory pathways, the development of the asthma phenotype is likely to be related to a complex interplay of a large number of genes combined with environmental factors.

Eosinophils are thought to be key effector cells in asthma by the release of basic granule proteins, membrane phospholipid metabolites and a variety of cytokines. For example, the eosinophil basic proteins have been found to be highly toxic in vitro to respiratory epithelial cells, at concentrations detected in biological fluid from patients with asthma.

### Murine Ovalbumin Study

Eosinophils are white blood cells and one of the immune system components responsible for combating parasites and certain infections. Along with mast cells, they also control mechanisms associated with allergy and asthma. The presence of eosinophils in the gut, lung or skin is associated with disease. Eosinophils persist in the circulation for 8-12 hours, and can survive in tissue for an additional 8-12 days in the absence of stimulation. Eosinophils are important mediators of allergic responses and asthma pathogenesis and are associated with disease severity. Following activation. eosinophils release a range of powerful molecules including cationic granule proteins, reactive oxygen species, lipid mediators, enzymes, growth factors and cytokines. Many of the mediators released by eosinophils are toxic at high levels to host cells.

In order to understand if the Bifidobacterial strain and/or its' exopolysaccharide has the potential to directly influence lung inflammatory responses, particularly inflammatory responses mediated by eosinophils, we performed a murine ovalbumin (OVA) respiratory allergy study. Mice were sensitized to the protein OVA by intra peritoneal injection (with the adjuvant alum) on days 0, 14 and 21 followed by repeated daily OVA aerosol challenge on days 26-28. Animals were euthanized on day 29 for analysis of lung disease parameters. *Bifidobacterium longum* NCIMB 41003 (35624™) or the capsular exopolysaccharide was administered three times, via the nasal route, on days 19, 25 and 27.

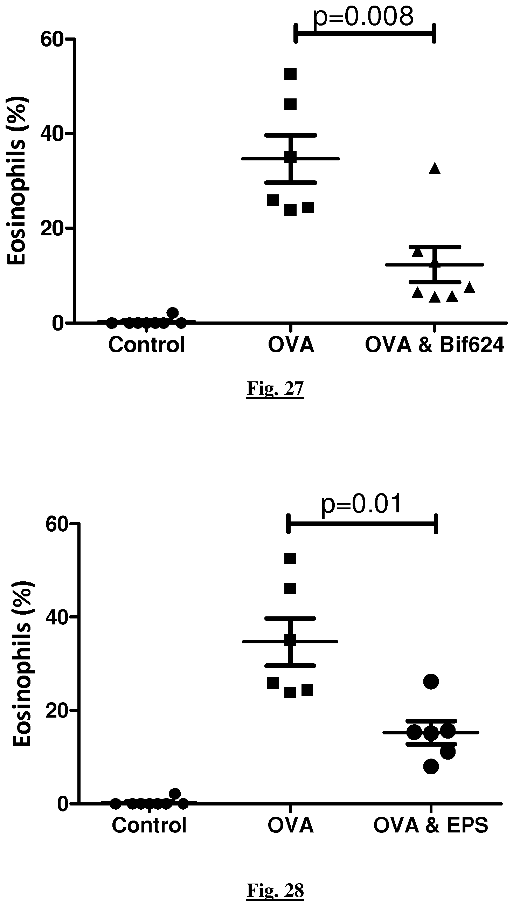

Surprisingly, exposure to the Bifidobacterial strain and its' exopolysaccharide significantly protected against inflammatory cell recruitment into the BAL of animals exposed to OVA, compared to animals that received OVA alone. When microscopic differential cell counts were performed, it was clear that the reduced BAL inflammatory cell count in bacterial treated (Fig. 27) or exopolysaccharide treated (Fig. 28) animals was primarily due to a reduced migration of eosinophils, which is the dominant infiltrating proinflammatory cell type in this murine model.

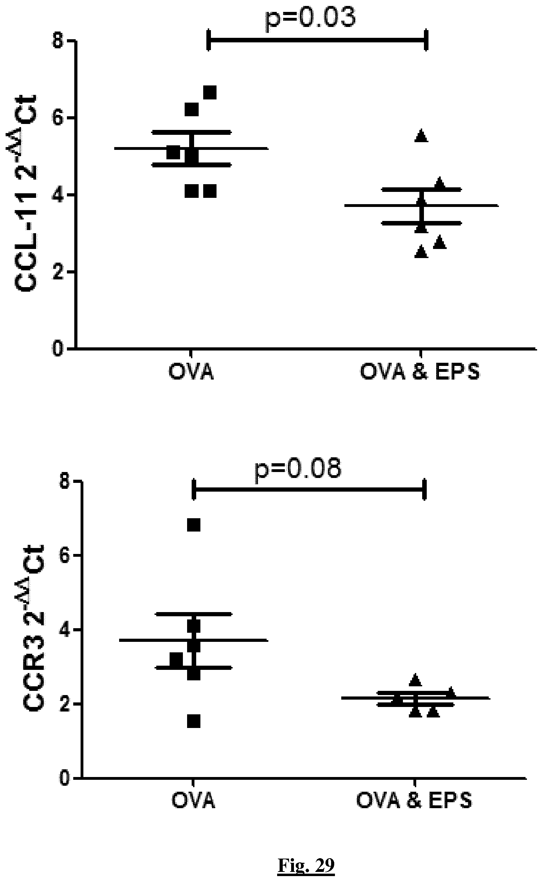

A number of factors are associated with eosinophil recruitment into the lung. C-C motif chemokine 11 (CCL11) gene expression was significantly reduced and CCR3 gene expression tended to be reduced in the exopolysaccharide treated lung (Fig. 29). CCL11, also known as eosinophil chemotactic protein and eotaxin-1 is a protein that in humans is encoded by the CCL11 gene. This gene is located on chromosome 17. CCL11 is a small cytokine belonging to the CC chemokine family. CCL11 selectively recruits eosinophils by inducing their chemotaxis, and therefore, is implicated in allergic responses (Garcia-Zepeda EA, Rothenberg ME, Ownbey RT, Celestin J, Leder P, Luster AD (Apr 1996). "Human eotaxin is a specific chemoattractant for eosinophil cells and provides a new mechanism to explain tissue eosinophilia". Nature Medicine. 2 (4): 449-56.). The effects of CCL11 are mediated by its binding to a G-protein-coupled receptor. Chemokine receptors for which CCL11 is a ligand include CCR2, CCR3 and CCR5. CCR3 binds and responds to a variety of chemokines, including eotaxin (CCL11), eotaxin-3 (CCL26), MCP-3 (CCL7), MCP-4 (CCL13), and RANTES (CCL5). It is highly expressed on eosinophils, and is also detected in TH1 and TH2 cells, as well as in airway epithelial cells. This receptor may contribute to the accumulation and activation of eosinophils and other inflammatory cells in the allergic airway, and possibly at sites of parasitic infection. It is also known to be an entry co-receptor for HIV-1.

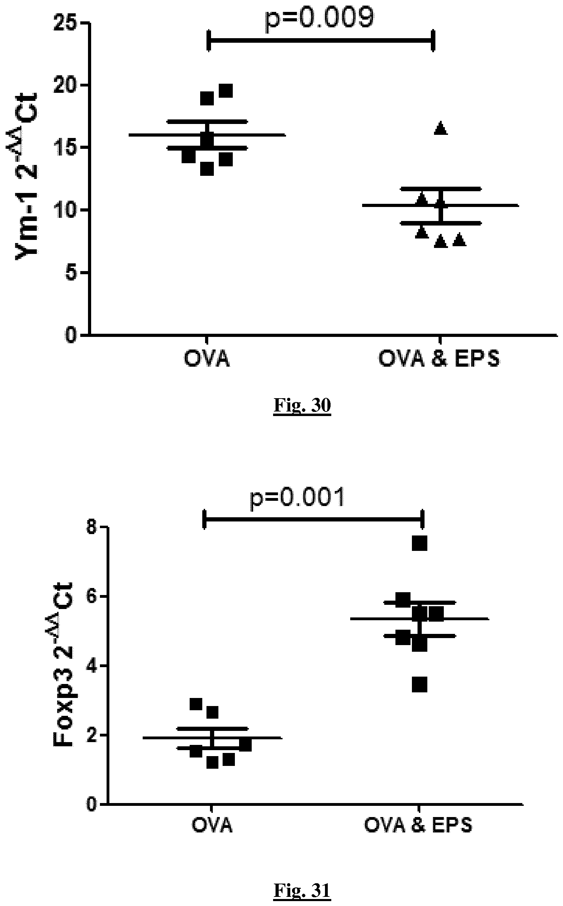

Alternative activation of alveolar macrophages is common in asthma, resulting in the M2 or alternatively activated phenotype characterized by decreased IL-12 production and the expression of specific factors such as Ym-1. In murine models of allergic airway disease, these M2 macrophages were identified as the source of eosinophil recruiting chemokines, which regulate migration of eosinophils from interstitial tissue into the airway lumen. Lung Ym-1 gene expression was significantly reduced following exopolysaccharide treatment (Fig. 30), suggesting that the exopolysaccharide may influence the M2 polarisation of macrophages resulting in fewer cells that secrete eosinophil recruiting chemokines.

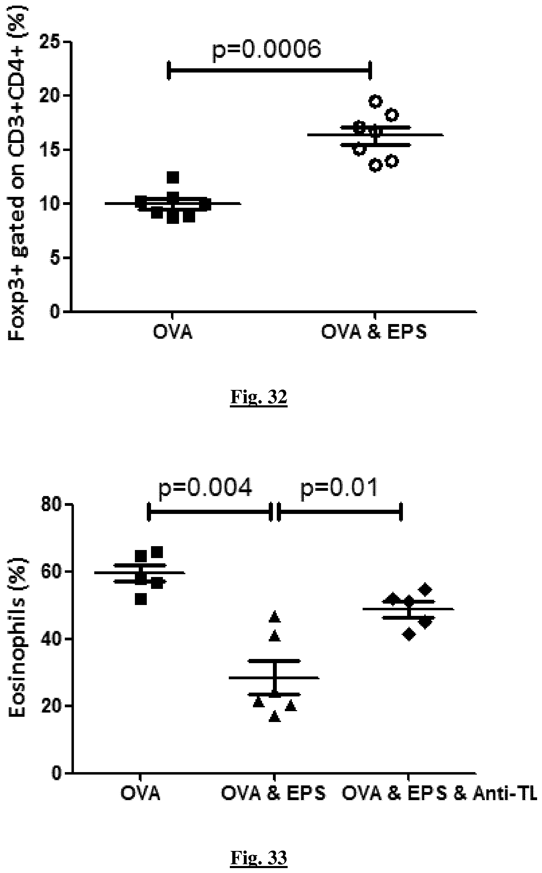

In order to further examine the potential mechanisms underpinning the protective effects of exopolysaccharide within the lung, the presence of regulatory T cells was quantified within lung tissue. Gene expression of the regulatory T cell transcription factor Foxp3 was significantly higher within the lung tissue of mice exposed to the exopolysaccharide (Fig. 31). In addition, single cell suspensions were generated from lung tissue and lymphocyte Foxp3 expression was quantified using flow cytometry. Consistent with the gene expression data, administration of the exopolysaccharide significantly increased the percentage of Foxp3+ lymphocytes within the murine lung (Fig. 32).

Foxp3+ regulatory T cells are potent suppressors of aberrant inflammatory immune responses. The primary mechanisms underpinning regulatory T cell effects include production of inhibitory cytokines (IL-10, TGF-□ and IL-35), effector cell cytolysis (via secretion of granzymes A and B), direct targeting of dendritic cells via inhibitory PD-1 and CTLA4 cell surface molecules, and metabolic disruption of effector cells (e.g. CD25, cAMP, adenosine, CD39, and CD73).

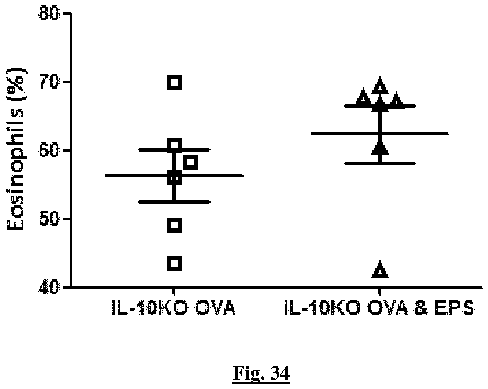

In vitro, TLR-2 was shown to recognise the exopolysaccharide and mediates the induction of the regulatory cytokine IL-10. The OVA respiratory allergy model was repeated using an anti-TLR-2 blocking antibody, which was administered into the lungs 1 hour before administration of the exopolysaccharide. When TLR-2 was blocked, the exopolysaccharide was significantly less effective in reducing the percentage of eosinophils in the lung lavages (Fig. 33). Similarly, in IL-10 knock-out mice, the exopolysaccharide no longer reduced eosinophil recruitment (Fig. 34). This data suggests that TLR-2 and IL-10 are both required to mediate the protective effects of the exopolysaccharide within the lung. The effect of both isolated EPS and EPS delivered through delivery of the whole strain demonstrates the potential utility of EPS in diseases characterised by airway inflammation such as Asthma and COPD.

## Claims

1. *Bifidobacterium longum* NCIMB 41003 for use in the prophylaxis or treatment of asthma, chronic obstructive pulmonary disease (COPD), eosinophilic COPD, infection-associated inflammation, allergen-induced inflammation, allergic rhinitis or chronic rhinosinusitis.

2. A polysaccharide comprising the structure 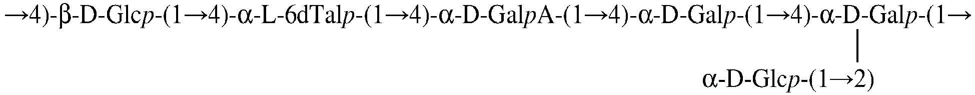 for use in the prophylaxis or treatment of asthma, chronic obstructive pulmonary disease (COPD), eosinophilic COPD, infection-associated inflammation, allergen-induced inflammation, allergic rhinitis or chronic rhinosinusitis.

3. A polysaccharide for use as claimed in claim 2 wherein at least one of the subunits is O-acetylated.

## Patentansprüche

1. *Bifidobacterium longum* NCIMB 41003 für die Verwendung bei der Prophylaxe oder Behandlung von Asthma, chronisch obstruktiver Lungenerkrankung (COPD), eosinophiler COPD, infektionsassoziierter Entzündung, allergeninduzierter Entzündung, allergischer Rhinitis oder chronischer Rhinosinusitis.

2. Polysaccharid, das die folgende Struktur umfasst: 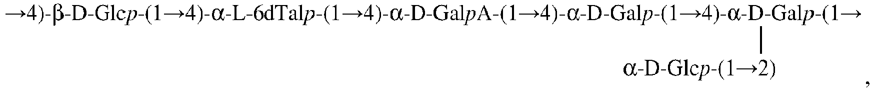 für die Verwendung bei der Prophylaxe oder Behandlung von Asthma, chronisch obstruktiver Lungenerkrankung (COPD), eosinophiler COPD, infektionsassoziierter Entzündung, allergeninduzierter Entzündung, allergischer Rhinitis oder chronischer Rhinosinusitis.

3. Polysaccharid für die Verwendung nach Anspruch 2, wobei mindestens eine der Untereinheiten O-acetyliert ist.

## Revendications

1. *Bifidobacterium longum* NCIMB 41003, pour une utilisation dans la prophylaxie ou le traitement de l'asthme, de la bronchopneumopathie chronique obstructive (BPCO), de la BPCO éosinophilique, des inflammations associées aux infections, des inflammations induites par des allergènes, de la rhinite allergique ou de la rhinosinusite chronique.

2. Polysaccharide, comprenant la structure 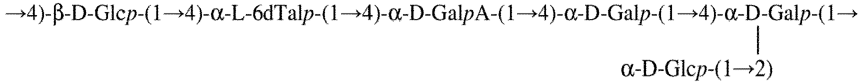 pour une utilisation dans la prophylaxie ou le traitement de l'asthme, de la bronchopneumopathie chronique obstructive (BPCO), de la BPCO éosinophilique, des inflammations associées aux infections, des inflammations induites par des allergènes, de la rhinite allergique ou de la rhinosinusite chronique.

3. Polysaccharide pour une utilisation selon la revendication 2, dans lequel au moins l'une des sous-unités est O-acétylée.
